# EUROPEAN PATENT SPECIFICATION

(11) **EP 3 943 011 B1**
(45) Date of publication and mention of the grant of the patent: **28.05.2025**
(21) Application number: 20777852.3
(22) Date of filing: 19.03.2020
(51) Int. Cl.: A61B 7/04, A61B 10/00, A61B 5/08, A61B 5/113, A61B 5/22, A61B 5/00

(54) **COMPUTER PROGRAM AND INFORMATION PROCESSING DEVICE FOR DETERMINING EXACERBATION OF CHRONIC OBSTRUCTIVE PULMONARY DISEASE**
COMPUTERPROGRAMM UND INFORMATIONSVERARBEITUNGSVORRICHTUNG ZUR BESTIMMUNG DER VERSCHLIMMERUNG DER CHRONISCH OBSTRUKTIVEN LUNGENERKRANKUNG
PROGRAMME INFORMATIQUE ET DISPOSITIF DE TRAITEMENT D'INFORMATIONS POUR DÉTERMINER L'AGGRAVATION DE LA MALADIE PULMONAIRE OBSTRUCTIVE CHRONIQUE

(30) Priority: 22.03.2019 JP 2019055536
(43) Date of publication of application: 26.01.2022
(73) Proprietor: Medical Optfellow Inc., Chofu-shi Tokyo 182-8585 (JP)
(72) Inventor: MATSUDA, Ken, Sendai-shi, Miyagi 980-0824 (JP)
(74) Representative: AWA Sweden AB
(86) International application number: PCT/JP2020/012464
(87) International publication number: WO 2020/196323

(56) References cited:
- WO-A2-2005/028029
- WO-A2-2017/032873
- JP-A- 2009 254 611
- JP-A- 2017 537 684
- JP-A- 2018 503 446
- JP-A- 2018 527 069
- US-A1- 2012 259 182
- US-A1- 2013 262 352
- US-A1- 2016 174 903
- US-A1- 2018 144 465
- AMAZON WEB SERVICES: "Amazon Machine Learning Developer Guide", 2 August 2016 (2016-08-02), XP055907246, Retrieved from the Internet <URL:https://docs.aws.amazon.com/machine-learning/latest/dg/machinelearning-dg.pdf> [retrieved on 20220330]
- WANG YU ET AL: "Respiratory electrophysiologic studies in chronic obstructive pulmonary disease", MEDICINE, vol. 98, no. 1, 1 January 2019 (2019-01-01), US, pages e13993, XP093225599, ISSN: 0025-7974, DOI: 10.1097/MD.0000000000013993
- VAN GEFFEN WOUTER ET AL: "Static and dynamic hyperinflation during severe acute exacerbations of chronic obstructive pulmonary disease", THE INTERNATIONAL JOURNAL OF CHRONIC OBSTRUCTIVE PULMONARY DISEASE, vol. Volume 13, 1 April 2018 (2018-04-01), pages 1269 - 1277, XP093225600, ISSN: 1178-2005, Retrieved from the Internet <URL:https://www.dovepress.com/getfile.php?fileID=41599> DOI: 10.2147/COPD.S154878

## Description

### [Technical Field]

The present invention relates to a program, and an information processing device for outputting information relating to exacerbation in COPD.

### [Background Art]

Since the past, there have been various detection techniques of diagnosing exacerbation in chronic obstructive pulmonary disease (COPD). For example, Patent Literature 1 discloses a method of detecting an exacerbation index of a COPD patient by measuring the expression level of IL-27 protein or its coding gene in a biological sample (such as, for example, blood or serum) derived from the COPD patient.

### [Citation List]

### [Patent Literature]

### [Patent Literature 1]

Japanese Published Unexamined Patent Application 2014-10117 involves taking a specimen from a user in order to determine exacerbation of COPD.

### [Further literature]

Document WO 2005/028029 A2 discloses a computer program and a device for classifying the origin of disordered breathing events and/or discriminating between disordered breathing events.

The relationship between lung hyperinflation and exacerbation of COPD is studied in, for instance, van Geffen WH, Kerstjens HA. Static and dynamic hyperinflation during severe acute exacerbations of chronic obstructive pulmonary disease. Int J Chron Obstruct Pulmon Dis. 2018 Apr 18;13:1269-1277. doi: 10.2147/COPD.S154878. PMID: 29713160; PMCID: PMC5912369.

Wang Y, Liu N, Zhang Z. Respiratory electrophysiologic studies in chronic obstructive pulmonary disease. Medicine (Baltimore). 2019 Jan;98(1):e13993. doi: 10.1097/MD.0000000000013993. PMID: 30608443; PMCID: PMC6344206 discuss the relationship between the action potential of the respiratory muscles and COPD.

### [Summary of Invention]

### [Technical Problem]

However, in the invention according to Patent Literature 1, a biological sample to be measured is collected from a COPD patient who undergoes exacerbation inspection, and thus there is concern of imposing a burden on the body of the COPD patient.

One aspect is to provide a program or the like capable of detecting an abnormality in a respiratory system disease without imposing a burden on the body of a patient.

### [Solution to Problem]

The invention is defined by the appended claims.

### [Advantageous Effects of Invention]

In one aspect, it is possible to detect an abnormality in a respiratory system disease without imposing a burden on the body of a patient.

### [Brief Description of Drawings]

Fig. 1 is a diagram illustrating an outline of a system that detects information relating to exacerbation in COPD.
Fig. 2 is a block diagram illustrating a configuration example of a server.
Fig. 3 is a diagram illustrating an example of a record layout of a patient DB.
Fig. 4 is a diagram illustrating an example of a record layout of a master DB.
Fig. 5 is a block diagram illustrating a configuration example of a terminal.
Fig. 6 is a block diagram illustrating a configuration example of a detection sensor.
Fig. 7 is a block diagram illustrating a configuration example of an electromyogram sensor.
Fig. 8A is an explanatory diagram illustrating motion information detected by the detection sensor.
Fig. 8B is an explanatory diagram illustrating the motion information detected by the detection sensor.
Fig. 9 is an explanatory diagram illustrating action potential information on the respiratory muscles of a healthy person detected by the electromyogram sensor.
Fig. 10A is an explanatory diagram illustrating action potential information on the respiratory muscles of a COPD patient at normal times detected by an electromyogram sensor 4.
Fig. 10B is an explanatory diagram illustrating action potential information on the respiratory muscles of the COPD patient during exacerbation detected by the electromyogram sensor 4.
Fig. 11 is an explanatory diagram illustrating an operation of a process of detecting information relating to exacerbation in COPD.
Fig. 12 is a flowchart illustrating a processing procedure of detecting the information relating to exacerbation in COPD.
Fig. 13 is a flowchart illustrating a processing procedure of a subroutine of a process of detecting an exacerbation level.
Fig. 14 is a block diagram illustrating a configuration example of a server of Embodiment 2.
Fig. 15 is a diagram illustrating a process of generating an exacerbation detection model.
Fig. 16 is a flowchart illustrating an example of a processing procedure of the process of generating the exacerbation detection model.
Fig. 17 is a diagram illustrating a process of detecting information relating to exacerbation using the exacerbation detection model.
Fig. 18 is a flowchart illustrating a processing procedure of detecting the information relating to exacerbation using the exacerbation detection model.
Fig. 19 is an explanatory diagram illustrating an operation of a process of detecting the information relating to exacerbation by adding a second sensor.
Fig. 20 is a flowchart illustrating a processing procedure of detecting the information relating to exacerbation by adding a microphone.
Fig. 21 is a block diagram illustrating a configuration example of a server of Embodiment 3.
Fig. 22 is a flowchart illustrating a processing procedure of detecting information relating to exacerbation using a second exacerbation detection model.
Fig. 23 is a flowchart illustrating an example of a processing procedure of a process of learning the second exacerbation detection model.
Fig. 24 is a block diagram illustrating a configuration example of a server of Embodiment 4.
Fig. 25 is a diagram illustrating an example of a record layout of a master DB of Embodiment 3.
Fig. 26 is a diagram illustrating an example of a record layout of an advice DB.
Fig. 27 is a flowchart illustrating a processing procedure of transmitting advice information according to activity content.
Fig. 28 is a flowchart illustrating a processing procedure of detecting information relating to exacerbation using a walking model.
Fig. 29 is a flowchart illustrating a processing procedure of changing a predetermined threshold of an electrical signal according to a probability value of exacerbation.
Fig. 30 is a block diagram illustrating a configuration example of a server of Embodiment 5.
Fig. 31 is a diagram illustrating an example of a record layout of a medication instruction information DB.
Fig. 32 is a diagram illustrating an example of a record layout of a treatment result DB.
Fig. 33 is a flowchart illustrating a processing procedure of transmitting medication instruction information according to information relating to exacerbation.
Fig. 34 is a flowchart illustrating a processing procedure of transmitting second advice information according to the information relating to exacerbation.

### [Description of Embodiments]

Hereinafter, the present invention will be described in detail with reference to the accompanying drawings illustrating embodiments thereof.

### (Embodiment 1)

Embodiment 1 relates to a mode of detecting an abnormality in a respiratory system disease on the basis of motion information relating to the motion of the respiratory muscles or the accessory respiratory muscles and action potential information relating to the respiratory muscles or the accessory respiratory muscles.

In the present embodiment, the motion information relating to the motion of the respiratory muscles or the accessory respiratory muscles is acquired using a detection sensor, and the action potential information relating to the respiratory muscles or the accessory respiratory muscles is acquired using an electromyogram sensor. The motion information includes information relating to the motion of the respiratory muscles or the accessory respiratory muscles, the motion of the thorax associated with the motion of the respiratory muscles or the accessory respiratory muscles, or the motion of the lungs or the diaphragm associated with their contraction and expansion. An abnormality in a respiratory system disease is detected on the basis of the acquired motion information and action potential information. In addition, in a case where an abnormality in a respiratory system disease is detected, the detected abnormality can be output to a patient or a doctor. Meanwhile, although an example of detecting information relating to exacerbation in COPD, which is one respiratory system disease, will be described below, abnormalities in other types of respiratory system diseases (for example, asthma, pneumonia, interstitial pneumonia, lung cancer, and the like) may be detected.

COPD is a disease in which toxic substances or gases (especially tobacco smoke) in the air cause chronic inflammation of the respiratory tract, resulting in airflow restriction (inadequate exhalation leads to inadequate ventilation) due to respiratory stenosis, destruction of the alveolar wall, and increased sputum. In addition, in COPD, the disease condition may rapidly worsen due to infection or the like, and this is called exacerbation. Normally, when exacerbation occurs, significant deterioration in the respiratory condition is observed, and even after recovery, it does not return to the respiratory condition before the exacerbation. Every time exacerbation recurs, the general physical condition and prognosis deteriorate.

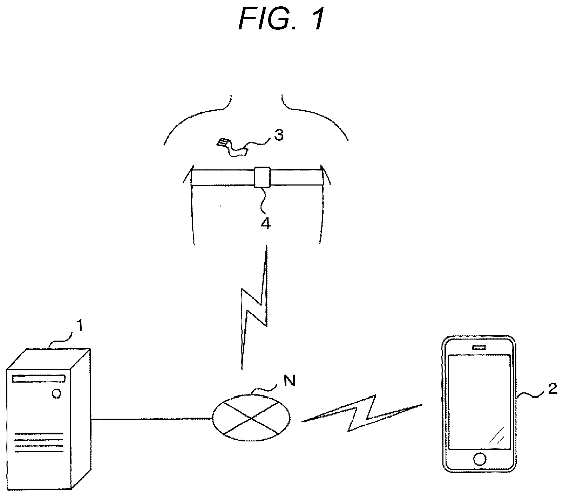

Fig. 1 is a diagram illustrating an outline of a system that detects information relating to exacerbation in COPD. The system of the present embodiment includes an information processing device 1, an information processing terminal 2, a detection sensor 3, and an electromyogram sensor 4, and each device transmits and receives information through a network N such as the Internet.

The information processing device 1 is an information processing device that processes, stores, transmits, and receives various types of information such as motion information relating to the motion of the respiratory muscles or the accessory respiratory muscles and action potential information relating to the respiratory muscles or the accessory respiratory muscles. The information processing device 1 is, for example, a server device, a personal computer, or the like. In the present embodiment, the information processing device 1 is assumed to be a server device, and is called a server 1 below for the purpose of simplification.

The information processing terminal 2 is a terminal device that performs reception, display, and the like of information on the detected abnormality in a respiratory system disease. The information processing terminal 2 is, for example, a smartphone, a cellular phone, a wearable device such as a wristwatch-type portable terminal, or an information processing instrument such as a tablet or a personal computer terminal. The information processing terminal 2 is called a terminal 2 below for the purpose of simplification.

The detection sensor 3 is a sensor that detects information on the motion of the respiratory muscles or the accessory respiratory muscles. The respiratory muscles are muscles used to expand and contract the thorax during respiration, and include the diaphragm, the internal intercostal muscles, the external intercostal muscles, and the like. The accessory respiratory muscles are a group of muscles including the sternocleidomastoid muscles, the anterior scalene muscles, the middle scalene muscles, the posterior scalene muscles, the serratus anterior muscles, the serratus posterior superior muscles, the serratus posterior inferior muscles, the latissimus dorsi muscles, the erector spinae muscles, the trapezius muscles, the quadratus lumborum muscles, the pectoralis major muscles, the pectoralis minor muscles, the rectus abdominis muscles, the internal oblique muscles, the external oblique muscles, the transverse abdominal muscles, and the like, and are used accessorily during respiration. For example, the detection sensor 3 having a belt shape or a tape shape is attached to the thorax of a patient to detect the movement of the respiratory muscles and the accessory respiratory muscles of the patient or the movement of the thorax based on the motion of the respiratory muscles and the accessory respiratory muscles.

As the detection sensor 3, for example, a piezoelectric element sensor, an extension sensor, a belt-shaped or tape-shaped tape sensor, an echo sensor, a bioelectric potential sensor that measures bioelectric impedance, or the like is used. Meanwhile, the detection sensor 3 is not limited to the above-described sensor, and may be, for example, a capacitance-type non-contact sensor capable of detecting the movement of the respiratory muscles and the accessory respiratory muscles, the movement of the thorax based on the motion of the respiratory muscles and the accessory respiratory muscles, or the respiration itself without touching a human body. The piezoelectric element sensor is a sensor that converts a force applied to a piezoelectric substance into a voltage, and may be, for example, a sensor for respiration monitoring that detects a respiration waveform using a piezoelectric element. For example, the above-described sensor for respiration monitoring (piezoelectric element sensor) is placed in the thorax which is a measurement region to convert a change in pressure applied to the thorax by the respiratory movement of the measurement region into a voltage, detect the movement of the respiratory muscles and the accessory respiratory muscles of the patient, and output the result as waveform data. Meanwhile, the above-described measurement region is not limited to the thorax, and may be, for example, the abdomen, the cervical region, the back, or the like. The echo sensor is, for example, a sensor that uses ultrasonic waves to perform liquid flow rate measurement, liquid identification, measurement of distance to an object, or the like. The echo sensor is used to detect the movement of the ribs, the diaphragm, the lungs, or the like. For example, the movement distance of the ribs or the movement distance of the diaphragm in respiration, information on its relaxation and contraction, the movement distance of the lungs, information on their expansion and contraction, or the like is detected. The bioelectric potential sensor indirectly detects the movement of the thorax and lungs from bioelectric impedance that changes depending on a change in the lung volume associated with respiration.

The extension sensor is a displacement sensor that expands and contracts like rubber, and monitors the movement of a subject in real time. The extension sensor is, for example, a thin sheet in which carbon nanotubes having a special structure and an elastomer material are layered. The extension sensor has elasticity like rubber along with conductivity, and its capacitance changes in accordance with the amount of extension and contraction. Since it is possible to make measurement from small strains to large strains, for example, a belt-shaped extension sensor wrapped around the thorax is mounted or a seal-shaped extension sensor is attached, and thus even small movements that have been difficult to measure so far such as changes of joints, the movement of muscles, the movement of the thorax during respiration, or a change in the vector of the movement of the thorax can be accurately measured. Meanwhile, the above-described measurement region is not limited to the thorax, and may be, for example, the abdomen, the back, or the like.

The electromyogram sensor 4 is a sensor that records the action potential of a muscle in a waveform. Depending on the characteristics of the recorded waveform, inclusive of the frequency, it is possible to acquire information relating to the diagnosis of the presence or absence of disorder, type, nature, region, and the like of nerves or muscles. The information relating to exacerbation in COPD can be detected by evaluating the respiratory muscles and the accessory respiratory muscles involved in COPD using the electromyogram sensor 4.

Meanwhile, although the detection sensor 3 and the electromyogram sensor 4 are shown separately in the drawing of the present embodiment, they may be configured integrally. In addition, in the present embodiment, an example in which the terminal 2, the detection sensor 3, and the electromyogram sensor 4 are separated from each other is shown, but there is no limitation thereto. For example, in a device configuration, the terminal 2, the detection sensor 3, and the electromyogram sensor 4 may all be integrated.

Next, a process of detecting the information relating to exacerbation in COPD will be described. The terminal 2 uses the detection sensor 3 to acquire the motion information relating to the motion of the respiratory muscles or the accessory respiratory muscles, and uses the electromyogram sensor 4 to acquire the action potential information relating to the respiratory muscles or the accessory respiratory muscles.

The motion information acquired by the detection sensor 3 includes, for example, the movement of the thorax due to the expansion and contraction of the respiratory muscles or the accessory respiratory muscles, a change in the vector of the movement of the thorax, the range of motion of the thorax, the speed of motion of the thorax, a stimulation time to the respiratory muscles, and the like. Meanwhile, although an example of acquiring the motion information using a piezoelectric element sensor (for example, a belt in which a piezoelectric element is incorporated), which is one type of detection sensor 3, will be described in the present embodiment, the same applies to other types of detection sensors 3 such as an extension sensor, a tape sensor, or a bioelectric potential sensor. In a case where a piezoelectric element sensor is attached to the thorax of a patient, the piezoelectric element sensor generates a voltage in accordance with the movement of the respiratory muscles or the accessory respiratory muscles of the patient, and a voltage (an operating electrical signal) is output from a piezoelectric element. Meanwhile, in a case where an extension sensor is used, a change in the speed or acceleration of expansion and contraction may be measured.

The action potential information acquired by the electromyogram sensor 4 is an action current signal which is generated with the contraction of the respiratory muscles or the accessory respiratory muscles during respiration, and also includes the duration of an action potential, a frequency waveform obtained by frequency-converting an action potential waveform, and the like.

The terminal 2 compares the acquired motion information and action potential information with each predetermined threshold, and in a case where a condition for detecting the information relating to exacerbation in COPD is satisfied, the terminal 2 detects the information relating to exacerbation. The information relating to exacerbation in COPD may be, for example, information indicating the presence or absence of COPD exacerbation, information on exacerbation levels classified in accordance with the probability value of exacerbation (for example, a value in the range of "0" to "1"), or the like. The probability value of exacerbation can be determined from information such as the respiratory condition of a COPD patient and the degree, frequency, and duration of dynamic hyperinflation (a disease condition in which the amount of residual air in the lungs increases with each breath due to air trapping associated with severe airflow obstruction) which is characteristically observed in the respiration of COPD patients during exacerbation. For example, this may be classified into four exacerbation levels (exacerbation level 1 to exacerbation level 4) in accordance with the probability value of exacerbation. Specifically, for example, in a case where the probability value of exacerbation is less than 0.05, the exacerbation level 1 (normal) indicating no exacerbation is determined. In a case where the probability value of exacerbation is equal to or greater than 0.05 and less than 0.5, the exacerbation level 2 indicating a low risk of exacerbation developing is determined. In a case where the probability value of exacerbation is equal to or greater than 0.5 and less than 0.8, the exacerbation level 3 indicating a high risk of exacerbation developing is determined. In a case where the probability value of exacerbation is equal to or greater than 0.8, the exacerbation level 4 indicating that exacerbation has occurred is determined.

Meanwhile, although a process of detecting information relating to exacerbation using the terminal 2 has been described in the present embodiment, there is no limitation thereto. For example, the server 1 may detect the information relating to exacerbation on the basis of the motion information and the action potential information. In this case, the server 1 transmits the detected information relating to exacerbation to the terminal 2.

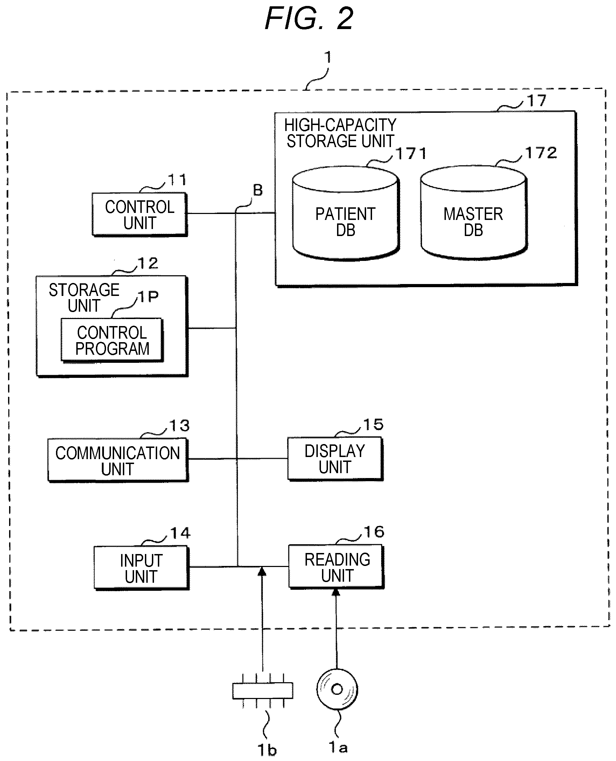

Fig. 2 is a block diagram illustrating a configuration example of the server 1. The server 1 includes a control unit 11, a storage unit 12, a communication unit 13, an input unit 14, a display unit 15, a reading unit 16, and a high-capacity storage unit 17.

The components are connected to each other through a bus B.

The control unit 11 includes arithmetic operation processing devices such as a central processing unit (CPU), a micro-processing unit (MPU), and a graphics processing unit (GPU), and performs various types of information processing, control processing, and the like related to the server 1 by reading out and executing a control program 1P stored in the storage unit 12. Meanwhile, although the control unit 11 is described as being a single processor in Fig. 2, it may be a multiprocessor.

The storage unit 12 includes memory elements such as a random access memory (RAM) and a read only memory (ROM), and stores the control program 1P required for the control unit 11 to execute a process. In addition, the storage unit 12 temporarily stores data or the like required for the control unit 11 to execute an arithmetic operation process. The communication unit 13 is a communication module for performing a process related to communication, and transmits and receives information to and from the terminal 2 or the like through the network N.

The input unit 14 is an input device such as a mouse, a keyboard, a touch panel, or a button, and outputs accepted operation information to the control unit 11. The display unit 15 is a liquid crystal display, an organic EL (electroluminescence) display, or the like, and displays various types of information in accordance with an instruction of the control unit 11.

The reading unit 16 reads a portable storage medium 1a including a compact disc (CD)-ROM or a digital versatile disc (DVD)-ROM. The control unit 11 may read the control program 1P from the portable storage medium 1a through the reading unit 16 and store the read program in the high-capacity storage unit 17. In addition, the control unit 11 may download the control program 1P from another computer through the network N or the like and store the downloaded program in the high-capacity storage unit 17. In addition, the control unit 11 may read the control program 1P from a semiconductor memory 1b.

The high-capacity storage unit 17 is, for example, a high-capacity storage device including a hard disk or the like. The high-capacity storage unit 17 includes a patient DB 171 and a master DB 172. The patient DB 171 stores information relating to a patient. The master DB 172 stores various types of thresholds for detecting the information relating to exacerbation in COPD.

Meanwhile, in the present embodiment, the storage unit 12 and the high-capacity storage unit 17 may be configured as an integrated storage device. In addition, the high-capacity storage unit 17 may be constituted by a plurality of storage devices. In addition, the high-capacity storage unit 17 may be an external storage device connected to the server 1.

Meanwhile, although the server 1 is described as being one information processing device in the present embodiment, it may be distributed and processed by a plurality of servers, or may be constituted by a virtual machine.

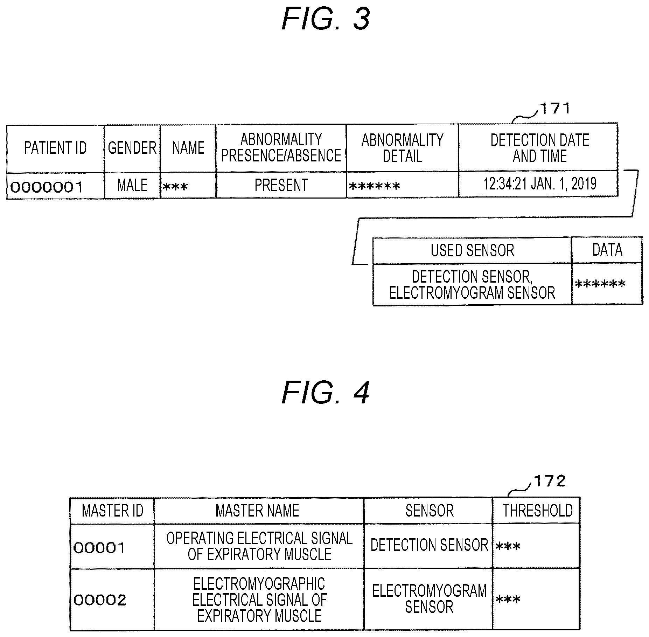

Fig. 3 is a diagram illustrating an example of a record layout of the patient DB 171. The patient DB 171 includes a patient ID column, a sex column, a name column, an abnormality presence/absence column, an abnormality detail column, a detection date and time column, a used sensor column, and a data column. The patient ID column stores the ID of a patient which is uniquely specified in order to identify each patient. The sex column stores the sex of the patient. The name column stores the name of the patient. The abnormality presence/absence column stores information indicating whether an abnormality has been detected for the patient. The abnormality detail column stores detailed information on the detected abnormality. The detection date and time column stores information on the date and time when the abnormality is detected. The used sensor column stores information on sensors used when the abnormality is detected. The data column stores sensor data detected by a used sensor. The sensor data is, for example, time-series data or the like.

Fig. 4 is a diagram illustrating an example of a record layout of the master DB 172. The master DB 172 includes a master ID column, a master name column, a sensor column, and a threshold column. The master ID column stores the ID of master data which is uniquely specified in order to identify each piece of master data. The master name column stores the name of the master data. The sensor column stores the name of a sensor to which a threshold is applied. The threshold column stores a reference value for comparison for detecting the information relating to exacerbation.

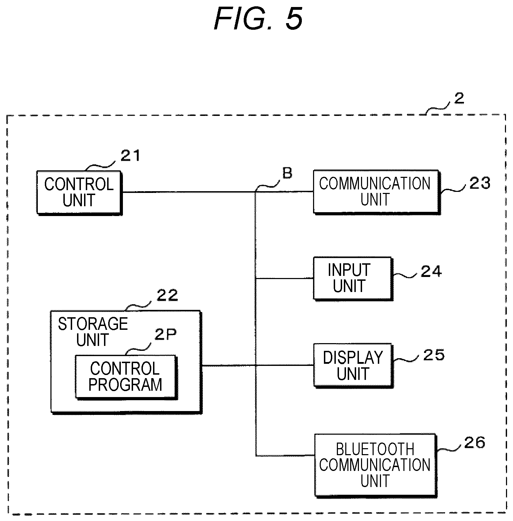

Fig. 5 is a block diagram illustrating a configuration example of the terminal 2. The terminal 2 includes a control unit 21, a storage unit 22, a communication unit 23, an input unit 24, a display unit 25, and a Bluetooth (registered trademark) communication unit 26. The components are connected to each other through a bus B.

The control unit 21 includes arithmetic operation processing devices such as a CPU and an MPU, and performs various types of information processing, control processing, and the like related to the terminal 2 by reading out and executing a control program 2P stored in the storage unit 22. Meanwhile, although the control unit 21 has been described as being a single processor in Fig. 5, it may be a multiprocessor. The storage unit 22 includes memory elements such as a RAM and a ROM, and stores the control program 2P required for the control unit 21 to execute a process. In addition, the storage unit 22 temporarily stores data or the like required for the control unit 21 to execute an arithmetic operation process.

The communication unit 23 is a communication module for performing a process related to communication, and transmits and receives information to and from the server 1 or the like through the network N. The input unit 24 may be a keyboard, a mouse, or a touch panel integrated with the display unit 25. The display unit 25 is a liquid crystal display, an organic EL display, or the like, and displays various types of information in accordance with an instruction of the control unit 21.

The Bluetooth communication unit 26 is a communication module for performing a process related to communication using Bluetooth, and transmits and receives information to and from the detection sensor 3, the electromyogram sensor 4, and the like. Meanwhile, although Bluetooth is shown as a near field communication means in Fig. 5, communication standards such as Zigbee or Wi-Fi may be used. In addition, wired communication may be adopted. Meanwhile, although an example of the Bluetooth communication unit 26 has been described in Fig. 5, there is no limitation thereto. For example, communication using a public network such as 5G or 4G may be performed.

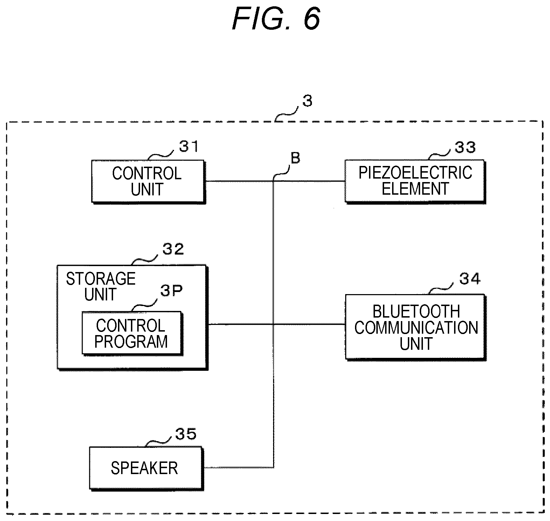

Fig. 6 is a block diagram illustrating a configuration example of the detection sensor 3. An example of using the above-described piezoelectric element sensor (for example, a belt or tape in which a piezoelectric element is incorporated) as the detection sensor 3 will be described here. The detection sensor 3 includes a control unit 31, a storage unit 32, a piezoelectric element 33, a Bluetooth communication unit 34, and a speaker 35. The components are connected to each other through a bus B.

The control unit 31 includes arithmetic operation processing devices such as a CPU and an MPU, and performs a control process or the like of converting a force applied to a piezoelectric substance read by the piezoelectric element 33 into a voltage by reading out and executing a control program 3P stored in the storage unit 32. The storage unit 32 includes memory elements such as a RAM and a ROM, and stores the control program 3P required for the control unit 31 to execute a process. In addition, the storage unit 32 temporarily stores data or the like required for the control unit 31 to execute an arithmetic operation process.

The piezoelectric element 33 is a passive element using a piezoelectric effect, and converts a force applied to a piezoelectric substance into a voltage. The Bluetooth communication unit 34 is a communication module for performing a process related to communication using Bluetooth, and transmits and receives voltage information (such as, for example, time-series data) to and from the terminal 2 or the like. Meanwhile, although Bluetooth is shown as a near field communication means in Fig. 6, communication standards such as Zigbee or Wi-Fi may be used. In addition, wired communication may be adopted. The speaker 35 is a device that converts an electrical signal into a sound. Meanwhile, the speaker 35 may be a headset connected to the detection sensor 3 using a short-range radio communication scheme such as Bluetooth.

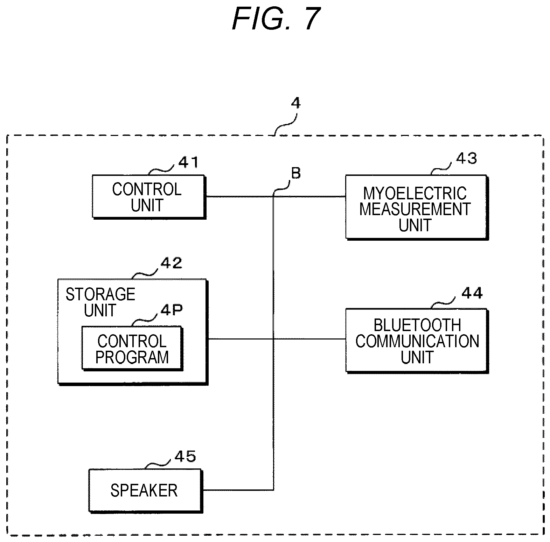

Fig. 7 is a block diagram illustrating a configuration example of the electromyogram sensor 4. The electromyogram sensor 4 includes a control unit 41, a storage unit 42, a myoelectric measurement unit 43, a Bluetooth communication unit 44, and a speaker 45. The components are connected to each other through a bus B.

The control unit 41 includes arithmetic operation processing devices such as a CPU and an MPU, and performs a control process or the like on the action potential information (myoelectric potential) read by the myoelectric measurement unit 43 by reading out and executing a control program 4P stored in the storage unit 42. The storage unit 42 includes memory elements such as a RAM and a ROM, and stores the control program 4P required for the control unit 41 to execute a process. In addition, the storage unit 42 temporarily stores data or the like required for the control unit 41 to execute an arithmetic operation process.

The myoelectric measurement unit 43 measures the action potential information relating to the respiratory muscles or the accessory respiratory muscles. The Bluetooth communication unit 44 is a communication module for performing a process related to communication using Bluetooth, and transmits and receives action potential information (such as, for example, time-series data) to and from the terminal 2 or the like. Meanwhile, in Fig. 7, Bluetooth is shown as a near field communication means, but communication standards such as Zigbee or Wi-Fi may be used. In addition, wired communication may be adopted. The speaker 45 is a device that converts an electrical signal into a sound. Meanwhile, the speaker 45 may be a headset connected to the electromyogram sensor 4 using a short-range radio communication scheme such as Bluetooth.

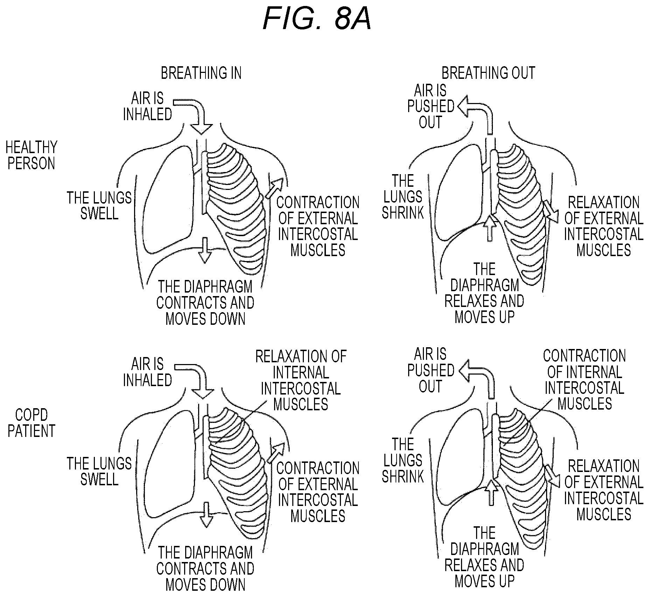

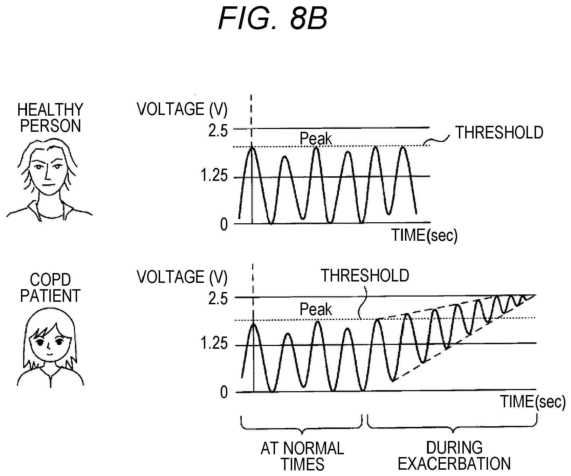

Figs. 8A and 8B are explanatory diagrams illustrating motion information detected by the detection sensor 3. Fig. 8A is an explanatory diagram illustrating respiratory physiology during normal respiration for a healthy person and a COPD patient. A person takes in oxygen into the body and expels carbon dioxide from the body through respiratory movement. The lungs are wrapped in the pleura and are located in the thorax. The lungs themselves have no force to expand and contract, but passively expand and contract through the mobility of the ribs forming the thorax and the expansion and contraction movement of the diaphragm or the like.

The diaphragm is the largest inspiratory muscle of the body, and is a muscle which is located around the fifth and sixth ribs, has a dome-like shape, and is used when inhaling. When a person inhales, that is, during inspiration, the inspiratory muscles including the diaphragm and the accessory inspiratory muscles contract in cooperation with each other, which expands the thorax and creates negative pressure in the thoracic cavity to inflate the lungs and take the outside air into the lungs. The inspiratory muscles and the accessory inspiratory muscles include the diaphragm, the external intercostal muscles, the sternocleidomastoid muscles, the anterior scalene muscles, the middle scalene muscles, the posterior scalene muscles, and the like.

When exhaling, that is, during expiration, these muscles stop contracting and relax. Since the lungs originally have the property of contracting by themselves like an inflated rubber balloon, the muscles that expand the thorax relax, and thus the lungs contract with their own contracting force to expel exhaled air. For this reason, respiration at rest in a healthy person does not require much activity of the expiratory muscles and the accessory expiratory muscles.

When a healthy person breathes (ventilates), he or she inhales the outside air by contracting the inspiratory muscles and the accessory inspiratory muscles to widen the thorax, and then expels the exhaled air by relaxing these muscles. However, a COPD patient has difficulty fully expelling the inhaled air due to ventilatory dysfunction in expiration associated with a prolonged expiration time. For this reason, the expulsion of the exhaled air is assisted by using the contraction of the expiratory muscles and the accessory expiratory muscles which are rarely used in a healthy person. The expiratory muscles and the accessory expiratory muscles include the internal intercostal muscles, the rectus abdominis muscles, the internal oblique muscles, the external oblique muscles, the transverse abdominal muscles, and the like.

Fig. 8B is an image diagram illustrating information on the motion of the respiratory muscles or the accessory respiratory muscles for a healthy person and a COPD patient. Fig. 8B shows waveform data of operating electrical signals (motion information) of the respiratory muscles or the accessory respiratory muscles. The horizontal axis represents time, and the unit is seconds (sec). The vertical axis represents a voltage, and the unit is volts (V).

The upper part of Fig. 8B is a graph illustrating a temporal change in a voltage which is output from the detection sensor 3 attached to a healthy person. In the case of the healthy person, it can be understood that the voltage periodically repeats the maximum and minimum with little temporal change. The lower part of Fig. 8B is a graph illustrating a temporal change in a voltage which is output from the detection sensor 3 attached to a COPD patient. In the case of the COPD patient, at normal times, the maximum and minimum with little temporal change are periodically repeated like the healthy person. However, in the case of exacerbation, temporal changes in the maximum and minimum continue to increase reflecting a progressive increase in the amount of residual air (dynamic hyperinflation) associated with exacerbation of ventilatory impairment during expiration.

In the present embodiment, as an example, in a case where a maximum change in a voltage which is output from the detection sensor 3 per unit time exceeds a predetermined threshold (for example, a slope of 0.2), it is determined that a first condition is satisfied. Specifically, the control unit 21 performs the following process. The control unit 21 acquires a voltage from the detection sensor 3 in a time-series manner. The control unit 21 obtains the maximum of the voltage in a time-series manner. The control unit 21 extracts the maximum of the voltage for a predetermined unit time (for example, 60 seconds), and calculates the slope of an approximate straight line formed by the extracted maximum. In a case where the control unit 21 determines that the slope exceeds a threshold determined in advance (for example, a slope of 0.2), it determines that the first condition is satisfied.

Meanwhile, the determination may of course be made using the minimum or both the maximum and minimum. Further, the determination may be made using a difference between the maximum and minimum. In addition, the control unit 21 may further determine that the first condition is satisfied, for example, in a case where the maximum mean value per unit time exceeds a threshold shown by the dotted line of Fig. 8B and the obtained slope exceeds the threshold. In addition, for example, the control unit 21 extracts the maximum and minimum of the voltage for a predetermined unit time (for example, 60 seconds), calculates the maximum slope of an approximate straight line formed by the extracted maximum, and calculates the minimum slope of an approximate straight line formed by the extracted minimum. The control unit 21 may determine that the first condition is satisfied in a case where it is determined that the maximum slope exceeds a maximum slope threshold determined in advance, the minimum slope exceeds a minimum slope threshold determined in advance, the minimum slope exceeds the maximum slope, and the duration exceeds a predetermined time (for example, 10 seconds).

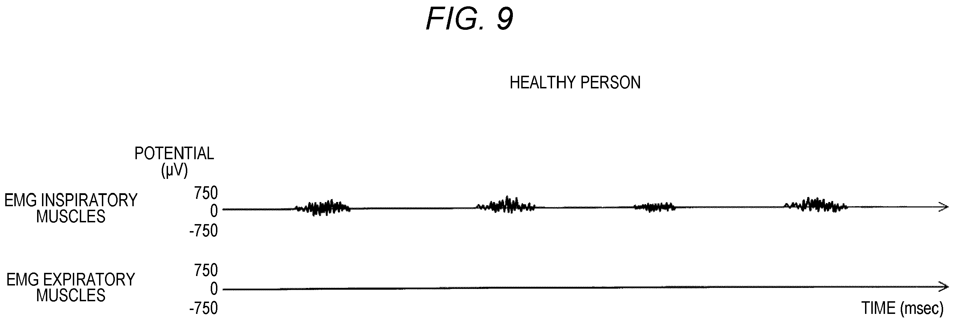

Fig. 9 is an explanatory diagram illustrating action potential information on the respiratory muscles of a healthy person detected by the electromyogram sensor 4. The horizontal axis represents time, and the unit is milliseconds (msec). The vertical axis represents a potential (action potential), and the unit is microvolts (µV). The upper part of the graph in Fig. 9 represents a temporal change in the potential of the inspiratory muscles of a healthy person. For the inspiratory muscles, a potential waveform having an amplitude of approximately 100 µV is confirmed at regular intervals. The lower part of the graph in Fig. 9 represents a temporal change in the potential of the expiratory muscles of the healthy person. As is clear from Fig. 9, the expiratory muscles of the healthy person are not very active, and thus the corresponding potential waveform is also minute and can hardly be confirmed.

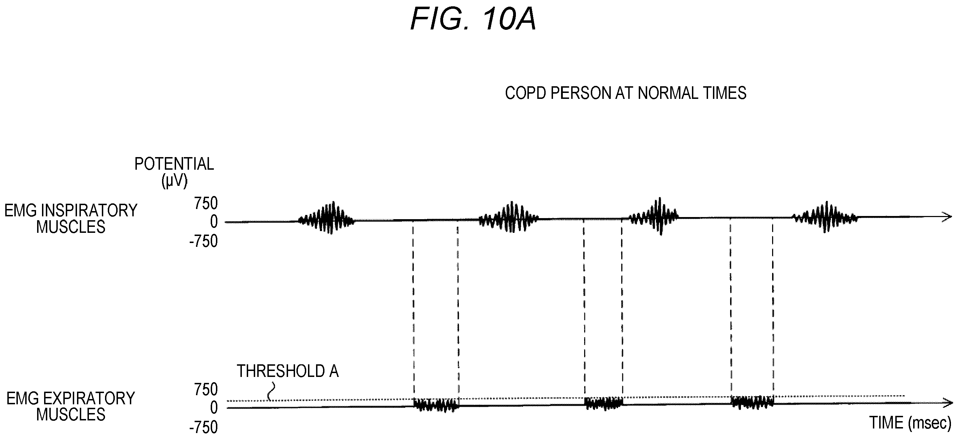

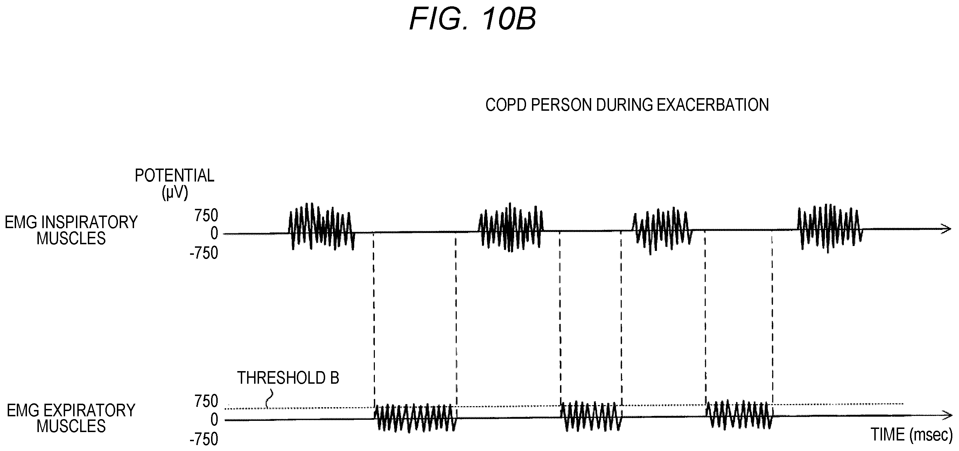

Figs. 10A and 10B are explanatory diagrams illustrating action potential information on the respiratory muscles of a COPD patient detected by the electromyogram sensor 4. Meanwhile, the horizontal axis and the vertical axis are the same as those in Fig. 9, and thus description thereof will not be given.

Fig. 10A is an explanatory diagram illustrating action potential information on the respiratory muscles of the COPD patient at normal times. In the COPD patient, since the activity of the inspiratory muscles at normal times becomes stronger than the activity of the inspiratory muscles of the healthy person (at normal times) in Fig. 9, the amplitude of the action potential of the inspiratory muscles of the COPD patient becomes larger than the amplitude of the action potential of the inspiratory muscles of the healthy person. In addition, in the COPD patient, since the expiratory muscles contract even in expiration at normal times, the amplitude of the action potential of the expiratory muscles is larger than that in Fig. 9.

Fig. 10B is an explanatory diagram illustrating action potential information on the respiratory muscles of the COPD patient during exacerbation. In a case where exacerbation occurs, the COPD patient attempts to take the outside air into the lungs by further contracting the inspiratory muscles to widen the thorax. Thereby, the inspiratory muscles contract more strongly than at normal times, and the amplitude of the action potential of the inspiratory muscles during exacerbation becomes larger than the amplitude of the action potential of the inspiratory muscles at normal times. In addition, the duration of the action potential of the inspiratory muscles during exacerbation becomes shorter than the duration of the action potential of the inspiratory muscles at normal times. Further, when these signals are analyzed with a Fourier transformation, the frequency distribution in a case where exacerbation occurs is different from that at normal times.

The COPD patient has increased ventilatory dysfunction in expiration associated with a prolonged expiration time in a case where exacerbation occurs. For this reason, the expiratory muscles are required to contract more strongly than normal in order to support ventilation. Thereby, the amplitude of the action potential of the expiratory muscles during exacerbation becomes larger than the amplitude of the action potential of the expiratory muscles at normal times. In addition, since it takes time to expel the exhaled air, the duration of the action potential of the expiratory muscles during exacerbation also becomes longer than the duration of the action potential of the expiratory muscles at normal times. Further, when these signals are analyzed with a Fourier transformation, the frequency distribution in a case where exacerbation occurs is different from that at normal times.

The dotted lines in Fig. 10 indicate predetermined thresholds of the action potential of the expiratory muscles at normal times and during exacerbation. It is possible to detect exacerbation by comparing the peak value of the action potential of the expiratory muscles of the COPD patient with the threshold of the action potential of the expiratory muscles at normal times and during exacerbation.

For example, the COPD patient may be provided with thresholds of action potentials at normal times and during exacerbation, and exacerbation levels may be defined in accordance with the provided thresholds. Specifically, the control unit 21 of the terminal 2 compares the peak value of the action potential of the expiratory muscles detected by the electromyogram sensor 4 with the thresholds of the action potentials at normal times and during exacerbation.

In a case where the control unit 21 determines that the peak value of the action potential of the expiratory muscles is less than a threshold A of the action potential of the expiratory muscles at normal times, the exacerbation level 2 indicating a low risk of developing exacerbation is determined. In a case where the control unit 21 determines that the action potential of the expiratory muscles is equal to or higher than the threshold A of the action potential of the expiratory muscles at normal times and is less than a threshold B (for example, a value increased by 30% of the threshold A) of the action potential of the expiratory muscles during exacerbation, the exacerbation level 3 indicating a high risk of exacerbation developing is determined.

In a case where the control unit 21 determines that the peak value of the action potential of the expiratory muscles is equal to or higher than the threshold B of the action potential of the expiratory muscles during exacerbation, the exacerbation level 4 indicating that exacerbation has occurred is determined. For example, in a case where the exacerbation level 4 is determined, the control unit 21 may display information relating to exacerbation on the display unit 25.

Meanwhile, the peak values of all the action potentials are not limited to being equal to or higher than a predetermined threshold. For example, the terminal 2 may determine the exacerbation level by comparing the number of times the peak value of the action potential per unit time is equal to or higher than the predetermined threshold with a predetermined number of times.

In addition, the exacerbation level may be determined according to the duration of the action potential of the expiratory muscles. For example, the COPD patient may be provided with thresholds of the duration of action potentials at normal times and during exacerbation, and it may be determined whether exacerbation has occurred in accordance with the provided thresholds of the duration.

Specifically, in a case where the control unit 21 determines that the duration of the action potential of the expiratory muscles is less than the threshold of the duration of the action potential at normal times, the exacerbation level 2 indicating a low risk of exacerbation developing is determined. In a case where the control unit 21 determines that the duration of the action potential of the expiratory muscles is equal to or higher than the threshold of the duration of the action potential at normal times and is less than the threshold of the duration of the action potential during exacerbation, the exacerbation level 3 indicating a high risk of exacerbation developing is determined. In a case where the control unit 21 determines that the duration of the action potential of the expiratory muscles is equal to or higher than the threshold of the duration of the action potential of the expiratory muscles during exacerbation, the exacerbation level 4 indicating that exacerbation has occurred is determined.

In addition, the exacerbation level may be determined on the basis of both the threshold of the action potential of the expiratory muscles and the threshold of the duration. For example, in a case where the control unit 21 determines that the peak value of the action potential of the expiratory muscles is equal to or higher than the threshold of the action potential during exacerbation and the duration of the action potential of the expiratory muscles is equal to or higher than the threshold of the duration of the action potential of the expiratory muscles during exacerbation, the exacerbation level 4 indicating that exacerbation has occurred may be determined.

In addition, the exacerbation level may be determined according to the frequency distribution of the action potential of the expiratory muscles. For example, the COPD patient may be provided with thresholds of the mean power frequency or the median power frequency at normal times and during exacerbation, and it may be determined whether exacerbation has occurred in accordance with the provided thresholds of the mean power frequency or the median power frequency.

Meanwhile, there is no limitation to the process of determining the exacerbation level described above. For example, the exacerbation level may be determined using the action potential information of both the inspiratory muscles and the expiratory muscles or only the action potential information of the inspiratory muscles.

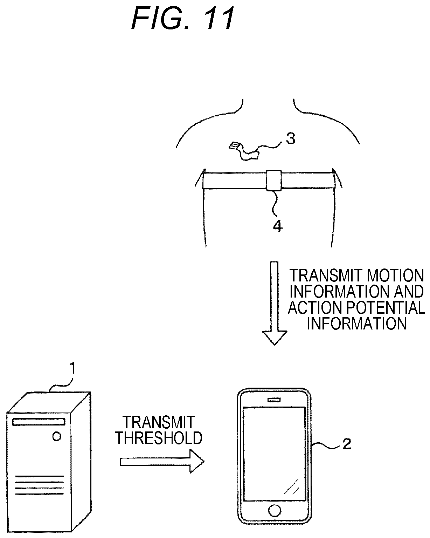

Fig. 11 is an explanatory diagram illustrating an operation of a process of detecting information relating to exacerbation in COPD. The control unit 21 of the terminal 2 acquires motion information relating to the motion of the respiratory muscles or the accessory respiratory muscles from the detection sensor 3 and action potential information relating to the respiratory muscles or the accessory respiratory muscles from the electromyogram sensor 4 through the Bluetooth communication unit 26. The motion information and the action potential information are time-series data measured for each predetermined unit time (such as, for example, every minute or second). In addition, the motion information and the action potential information may be time-series waveform images. In addition, the motion information and the action potential information may be frequency characteristic graphs. Specifically, frequency analysis is performed from the time-series data of the motion information and the action potential information, and fluctuating waves corresponding to the movement of the thorax and the action potentials of the respiratory muscles and the accessory respiratory muscles are extracted. For frequency analysis, for example, discrete Fourier transformation (DFT) performed on a discretized signal may be used. The control unit 21 may store and centrally manage the acquired motion information and action potential information using, for example, time-series data infrastructure.

The control unit 21 acquires various types of thresholds for detecting the information relating to exacerbation in COPD from the master DB 172 of the high-capacity storage unit 17 of the server 1 through the communication unit 23. Meanwhile, various types of thresholds for detecting the information relating to exacerbation in COPD may be acquired from the server 1 in advance, or may be stored in advance in the storage unit 22 of the terminal 2. For example, a predetermined threshold of the operating electrical signal of the respiratory muscles, predetermined thresholds of the electrical signal of the electromyogram related to the expiratory muscles at normal times and during exacerbation, predetermined thresholds of the duration of the electrical signal of the expiratory muscles at normal times and during exacerbation, and the like are acquired.

The control unit 21 determines whether the first condition is satisfied on the basis of the motion information detected by the detection sensor 3. In a case where the control unit 21 determines that the first condition is satisfied, the control unit 21 further detects information relating to exacerbation on the basis of the action potential information relating to the expiratory muscles and the predetermined threshold acquired by the electromyogram sensor 4. The control unit 21 displays the detected information relating to exacerbation on the display unit 25.

Meanwhile, in a case where the information relating to exacerbation is detected, the control unit 21 may transmit the effect of exacerbation detection to the detection sensor 3 and the electromyogram sensor 4 through the Bluetooth communication unit 26. For example, the control unit 31 of the detection sensor 3 may receive the effect of exacerbation detection transmitted from the terminal 2, and notify the COPD patient with an alarm, voice information, or the like through the speaker 35. The control unit 41 of the electromyogram sensor 4 may receive the effect of exacerbation detection transmitted from the terminal 2, and notify the COPD patient with an alarm, voice information, or the like through the speaker 45.

Meanwhile, although an example of the action potential information relating to the expiratory muscles has been described in the above-described process, there is no limitation thereto. For example, the information relating to exacerbation may be detected on the basis of the action potential information relating to the inspiratory muscles or the action potential information relating to both the inspiratory muscles and the expiratory muscles.

The control unit 21 transmits the detected information relating to exacerbation to the server 1 through the communication unit 23. The control unit 11 of the server 1 receives the information relating to exacerbation transmitted from the terminal 2 through the communication unit 13, and stores the received information relating to exacerbation in the patient DB 171 of the high-capacity storage unit 17. Meanwhile, there is no limitation to the process of detecting the information relating to exacerbation described above. For example, the terminal 2 may detect the information relating to exacerbation on the basis of the range of motion of the thorax, the speed of motion of the thorax, the stimulation time to the respiratory muscles, the frequency change of the action potential of the respiratory muscles, and the like acquired by the detection sensor 3 and the electromyogram sensor 4.

During muscle fatigue, the amplitude of an electromyogram signal increases, which leads to wave slowing in which the frequency component moves from a high-frequency region to a low-frequency region. The wave slowing is a sign of exacerbation. Hereinafter, an example of the frequency change of the action potential of the respiratory muscles will be described. The terminal 2 measures the power spectrum of the electromyogram signal of the respiratory muscles and performs a Fourier transformation. The power spectrum is obtained by converting the electromyogram waveform as the distribution of frequency components on the horizontal axis and the square of the amplitude of each frequency component (signal power) on the vertical axis.

The terminal 2 calculates a change value of the mean power frequency (MPF) or the median power frequency (MF) as an index indicating the characteristics of the power distribution of the spectrum. The mean power frequency is a mean value of each frequency. The median power frequency is a frequency that divides the area of the power spectrum into two equal areas. The terminal 2 determines whether there is a tendency of wave slowing on the basis of the calculated change value of the mean power frequency or the median power frequency. For example, in the case of a gradual transition from a high-frequency band (for example, 86.4 Hz) to a low-frequency band (for example, 67.4 Hz), the terminal 2 calculates the change value of the mean power frequency or the median power frequency. In a case where it is determined that the calculated change value is equal to or higher than a predetermined threshold, the terminal 2 outputs the information relating to exacerbation. Meanwhile, the exacerbation level may be output in accordance with each of a plurality of provided thresholds. For example, the exacerbation level 3 may be set in a case where the change value is equal to or higher than a first threshold and less than a second threshold, and the exacerbation level 4 may be set in a case where the change value is equal to or higher than the second threshold. In addition, in the electromyogram signal in which frequency analysis is performed, all data including that of the expiratory muscles and the inspiratory muscles may be used, or data of either the expiratory muscles or the inspiratory muscles may be used.

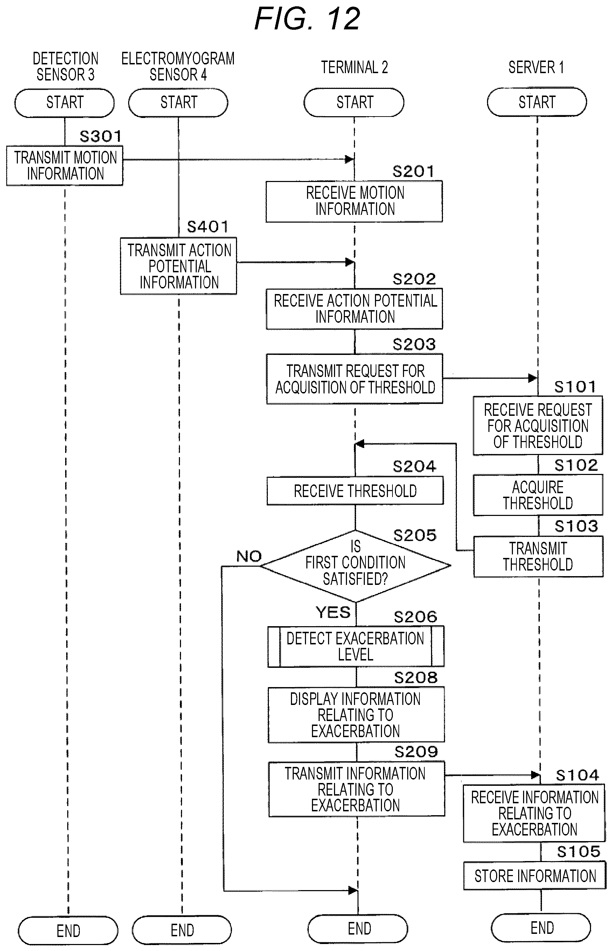

Fig. 12 is a flowchart illustrating a processing procedure of detecting the information relating to exacerbation in COPD. The control unit 31 of the detection sensor 3 transmits the motion information relating to the motion of the respiratory muscles or the accessory respiratory muscles to the terminal 2 through the Bluetooth communication unit 34 (step S301). The control unit 21 of the terminal 2 receives the motion information transmitted from the detection sensor 3 through the Bluetooth communication unit 26 (step S201). The control unit 41 of the electromyogram sensor 4 transmits the action potential information relating to the respiratory muscles or the accessory respiratory muscles to the terminal 2 through the Bluetooth communication unit 44 (step S401). The control unit 21 of the terminal 2 receives the action potential information transmitted from the electromyogram sensor 4 through the Bluetooth communication unit 26 (step S202).

The control unit 21 transmits a request for acquiring various types of thresholds for detecting the information relating to exacerbation in COPD to the server 1 through the communication unit 23 (step S203). The control unit 11 of the server 1 receives the request for the acquisition of the thresholds transmitted from the terminal 2 through the communication unit 13 (step S101). The control unit 11 acquires various types of thresholds for detecting the information relating to exacerbation in COPD from the master DB 172 of the high-capacity storage unit 17 in accordance with the received request for the acquisition of the thresholds (step S102). The control unit 11 transmits various types of acquired thresholds to the terminal 2 through the communication unit 13 (step S103).

The control unit 21 of the terminal 2 receives various types of thresholds transmitted from the server 1 through the communication unit 23 (step S204). The control unit 21 determines whether the first condition is satisfied on the basis of the received threshold and motion information (step S205). Specifically, for example, the control unit 21 acquires a voltage from the detection sensor 3 in a time-series manner, and obtains the maximum of the acquired voltage in a time-series manner. The control unit 21 extracts the maximum of the voltage within a predetermined unit time (for example, 60 seconds), and calculates the slope of an approximate straight line formed by the extracted maximum. In a case where the control unit 21 determines that the slope exceeds a threshold determined in advance, it determines that the first condition is satisfied.

Alternatively, for example, the control unit 21 extracts both the maximum and minimum of the voltage for a predetermined unit time, calculates the maximum slope of an approximate straight line formed by the extracted maximum, and calculates the minimum slope of an approximate straight line formed by the extracted minimum. In a case where it is determined that the maximum slope exceeds a maximum slope threshold determined in advance, the minimum slope exceeds a minimum slope threshold determined in advance, and the duration exceeds a predetermined time (for example, 10 seconds), the control unit 21 determines that the first condition is satisfied. Meanwhile, the minimum slope threshold is preferably set to a value larger than the maximum slope threshold.

In a case where it is determined that the first condition is not satisfied (NO in step S205), the control unit 21 ends the process. In a case where it is determined that the first condition is satisfied (YES in step S205), the control unit 21 executes a subroutine of a process of detecting the exacerbation level (step S206). Meanwhile, the subroutine of a process of detecting the exacerbation level will be described later. The control unit 21 displays the detected information relating to exacerbation (an exacerbation level) on the display unit 25 (step S208). The control unit 21 transmits the detected information relating to exacerbation to the server 1 through the communication unit 23 (step S209).

The control unit 11 of the server 1 receives the information relating to exacerbation transmitted from the terminal 2 through the communication unit 13 (step S104). The control unit 11 stores the received information relating to exacerbation in the patient DB 171 of the high-capacity storage unit 17 (step S105). Specifically, the control unit 11 stores a patient ID, a sex, a name, abnormality (exacerbation) presence/absence information, detailed information on the abnormality, detection date and time information, sensor information used when the abnormality is detected, and sensor data detected by a used sensor, as one record, in the patient DB 171.

Meanwhile, although an example in which the exacerbation level is further determined using the action potential information in a case where the first condition is satisfied has been described in the present embodiment, there is no limitation thereto. For example, even in a case where the first condition is not satisfied, the exacerbation level may be determined using the action potential information.

Meanwhile, although an example in which the above-described process is performed in the order of an exacerbation determination process (step S205) based on the motion information and an exacerbation determination process (step S206) based on the action potential information has been described, there is no limitation thereto. For example, the exacerbation determination process (step S206) based on the action potential information may be performed first, and then the exacerbation determination process (step S205) based on the motion information may be performed. Specifically, the control unit 21 of the terminal 2 detects the exacerbation level by executing a subroutine of a process of detecting the exacerbation level. For example, in a case where the exacerbation level 3 or higher indicating a high risk of exacerbation developing is determined, the control unit 21 determines whether the first condition is satisfied on the basis of the motion information. In a case where it is determined that the first condition is satisfied, the control unit 21 outputs the information relating to exacerbation.

Alternatively, in a case where the exacerbation determination process (step S205) based on the motion information and the exacerbation determination process (step S206) based on the action potential information are performed in parallel and the exacerbation determination conditions derived using both are satisfied, the information relating to exacerbation may be detected. Specifically, the control unit 21 of the terminal 2 determines whether the first condition is satisfied on the basis of the motion information. The control unit 21 detects the exacerbation level by executing a subroutine of a process of detecting the exacerbation level. For example, in a case where it is determined that the first condition is satisfied and the detected level is the exacerbation level 3 or higher indicating a high risk of exacerbation developing, the control unit 21 outputs the information relating to exacerbation.

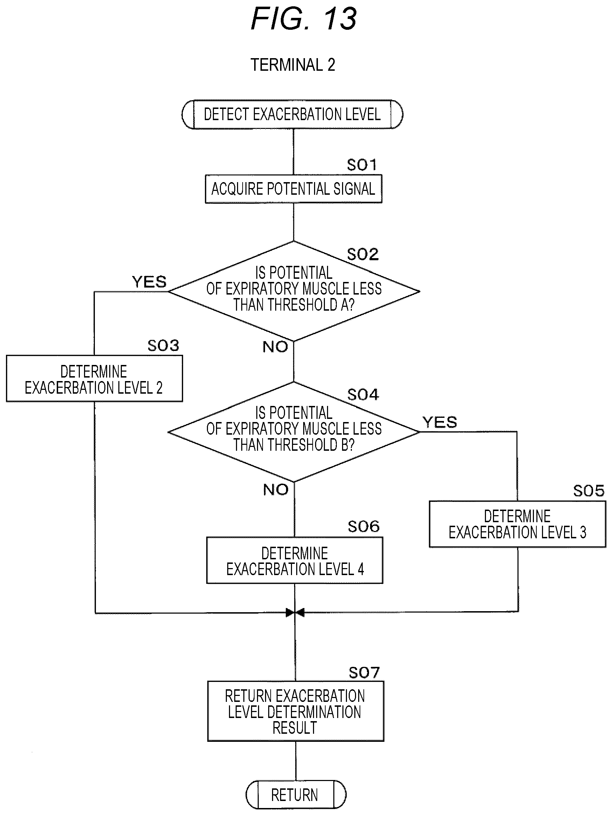

Fig. 13 is a flowchart illustrating a processing procedure of a subroutine of a process of detecting the exacerbation level. The control unit 21 of the terminal 2 acquires a potential signal acquired from the electromyogram sensor 4 (step S01). The control unit 21 detects a periodic change in the potential of the inspiratory muscles which is equal to or higher than a predetermined potential and exceeds a predetermined duration. The control unit 21 recognizes the periodic change in the potential of the inspiratory muscles, and then monitors a change in the potential of the expiratory muscles during the change in the potential of the inspiratory muscles. The control unit 21 detects the potential of the expiratory muscles during the change in the potential of the inspiratory muscles.

Meanwhile, the detection sensor 3 and the electromyogram sensor 4 may be used in combination to identify the potential of the inspiratory muscles or the potential of the expiratory muscles. For example, in a case where the detection sensor 3 is an extension sensor or a tape sensor, the tape stretches during inspiration, and thus the control unit 21 determines the potential signal acquired from the electromyogram sensor 4 as the potential of the inspiratory muscles at a timing from when the tape starts to stretch to its maximum stretch (MAX: elastic limit stretch). In contrast, since the tape starts to shrink during expiration, the control unit 21 determines the potential signal acquired from the electromyogram sensor 4 as the potential of the expiratory muscles at a timing from the maximum stretch (MAX) of the tape to its minimum stretch (MIN).

Alternatively, a learning model learned using training data including the feature amount of waveform data of the action potential acquired from the electromyogram sensor 4, a label indicating the potential of the inspiratory muscles, and a label indicating the potential of the expiratory muscles may be used to identify the potential of the inspiratory muscles or the potential of the expiratory muscles. In addition, the smaller of amplitude mean values of the action potential acquired from the electromyogram sensor 4 may be determined as the potential of the expiratory muscles.

The control unit 21 determines whether the peak potential of the expiratory muscles is less than the threshold A (the action potential of the expiratory muscles at normal times) (step S02). In a case where it is determined that the peak potential of the expiratory muscles is less than the threshold A (YES in step S02), the control unit 21 determines the exacerbation level 2 indicating a low risk of exacerbation developing (step S03). In a case where it is determined that the peak potential of the expiratory muscles is equal to or higher than the threshold A (NO in step S02), the control unit 21 determines whether the peak potential of the expiratory muscles is less than the threshold B (for example, a value increased by 30% of the threshold A) (step S04).

In a case where it is determined that the peak potential of the expiratory muscles is less than the threshold B (YES in step S04), the control unit 21 determines the exacerbation level 3 indicating a high risk of exacerbation developing (step S05). In a case where it is determined that the peak potential of the expiratory muscles is equal to or higher than the threshold B (NO in step S04), the control unit 21 determines the exacerbation level 4 indicating that exacerbation has occurred (step S06). The control unit 21 returns the determination result of the determined exacerbation level (step S07), ends the subroutine, and returns.

Meanwhile, although an example of the action potential information relating to the expiratory muscles has been described in the above-described process, there is no limitation thereto. For example, the exacerbation level may be detected on the basis of the action potential information relating to the inspiratory muscles or the action potential information relating to both the inspiratory muscles and the expiratory muscles.

In addition, although the peak potential of the action potential has been used in the present embodiment, there is no limitation thereto. For example, the duration of the action potential, the frequency distribution of the action potential, and the like may be used. An example in which the duration is used will be described below. The control unit 21 detects the start timing and the end timing shown by dotted lines in Fig. 10B on the basis of a change in the potential signal of the expiratory muscles. Specifically, the control unit 21 detects a timing at which temporal changes exceeding a predetermined threshold of the potential signal start to occur continuously as the start timing. In addition, the control unit 21 detects the start timing, and then detects a timing at which temporal changes that do not exceed the predetermined threshold of the potential signal start to occur continuously as the end timing. The control unit 21 outputs the information relating to exacerbation in a case where the time from the start timing to the end timing exceeds a threshold determined in advance. Meanwhile, a plurality of thresholds may be provided, the exacerbation level may be set to 3 in a case where these thresholds are equal to or higher than the first threshold and less than the second threshold, and the exacerbation level may be set to 4 in a case where these thresholds are equal to or higher than the second threshold.

According to the present embodiment, an abnormality in a respiratory system disease can be detected on the basis of the motion information acquired by the detection sensor 3 and the action potential information acquired by the electromyogram sensor 4. This makes it possible to reduce a burden on a patient's body without collecting a biological sample (such as, for example, blood or serum) from a COPD patient who undergoes exacerbation inspection.

### (Embodiment 2)

Embodiment 2 relates to a mode of detecting information relating to exacerbation in COPD using an exacerbation detection model constructed by deep learning. Meanwhile, content overlapping that of Embodiment 1 will not be described.

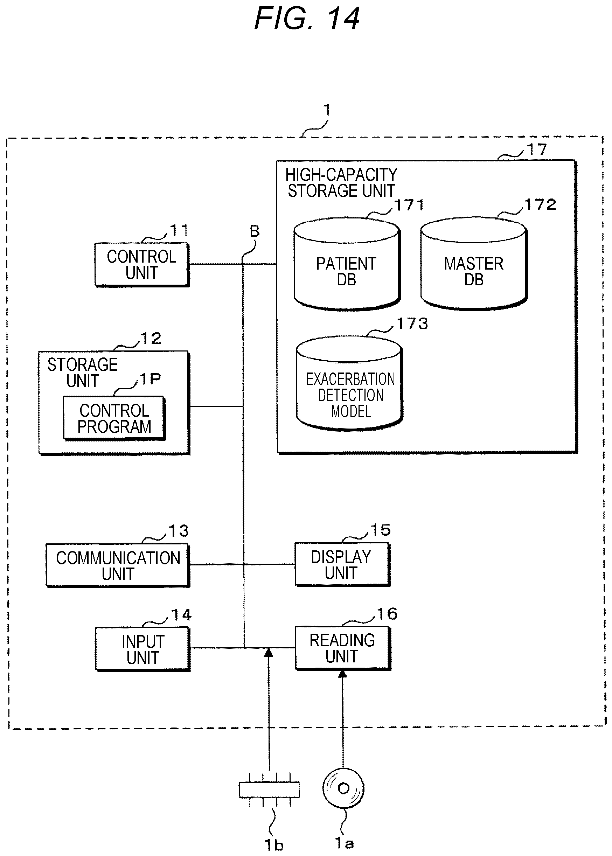

Fig. 14 is a block diagram illustrating a configuration example of a server 1 of Embodiment 2. Meanwhile, content overlapping that of Fig. 2 is denoted by the same reference numerals and signs and thus description thereof will be omitted. The high-capacity storage unit 17 includes an exacerbation detection model 173. The exacerbation detection model 173 is a detector that detects information relating to exacerbation in COPD, and is a learned model generated by machine learning. The learned model is used as a program module which is a portion of artificial intelligence software. Hereinafter, a process of detecting information relating to exacerbation will be described using the exacerbation detection model 173 constructed by deep learning.

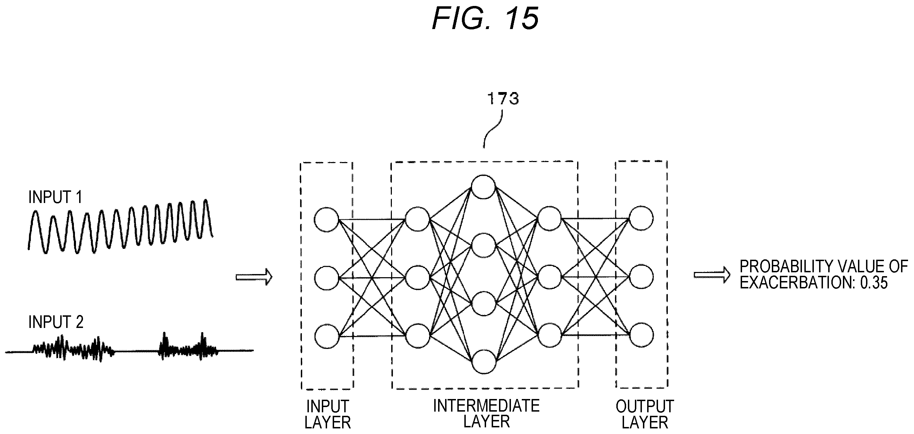

Fig. 15 is a diagram illustrating a process of generating the exacerbation detection model 173. Fig. 15 conceptually illustrates a process of generating the exacerbation detection model 173 by performing machine learning. The process of generating the exacerbation detection model 173 will be described with reference to Fig. 15.

The server 1 in the present embodiment constructs (generates) a neural network, as the exacerbation detection model 173, in which a waveform image is used as an input and information indicating the presence or absence of the information relating to exacerbation in COPD is used as an output by performing deep learning to learn the feature amount of the waveform image of an exacerbation point within an image of an electrical signal relating to motion information acquired from the detection sensor 3 and an image of action potential information acquired from the electromyogram sensor 4. Deep learning may be performed on an image after frequency analysis. The neural network is, for example, a convolutional neural network (CNN), and has an input layer that accepts input of a waveform image, an output layer that outputs information (an identification result) indicating the presence or absence of an abnormality, and an intermediate layer that extracts an image feature amount of the waveform image.

The input layer has a plurality of neurons that accept input of the pixel value of each pixel included in a waveform image, and transfers the input pixel value to the intermediate layer. The intermediate layer has a plurality of neurons that extract the image feature amount of a waveform image, and transfers the extracted image feature amount to the output layer. For example, in a case where the exacerbation detection model 173 is a CNN, the intermediate layer has a configuration in which a convolution layer that convolves the pixel values of pixels input from the input layer and a pooling layer that maps the pixel values convolved in the convolution layer are alternately connected to each other, and finally extracts the feature amount of the image while compressing pixel information of the waveform image. The output layer has one or a plurality of neurons that output identification results of identifying exacerbation points in COPD, and identifies the presence or absence of information relating to exacerbation on the basis of the image feature amount output from the intermediate layer.

Meanwhile, although the exacerbation detection model 173 has been described as being a CNN in the present embodiment, the exacerbation detection model 173 is not limited to a CNN, and may be a learned model constructed by other learning algorithms such as a neural network other than the CNN, a support vector machine (SVM), a Bayesian network, or a regression tree.

As shown in Fig. 15, for example, an input 1 is a waveform image of information on the motion of the respiratory muscles for a predetermined time (for example, 60 seconds) detected by the detection sensor 3, and an input 2 is a waveform image of action potential information relating to the respiratory muscles for a predetermined time (for example, 60 seconds) detected by the electromyogram sensor 4. The waveform image of the motion information (input 1) and the waveform image of the action potential information (input 2) are waveform images indicating signals at the same timing. The control unit 11 of the server 1 detects and outputs the information relating to exacerbation from the waveform images of the input motion information and action potential information in accordance with a command from the learned exacerbation detection model 173 stored in a memory.

The output information relating to exacerbation may be, for example, information on the presence or absence of exacerbation, the probability value of exacerbation, an exacerbation level classified in accordance with the probability value of exacerbation, or the like. The waveform image (input 2) of the action potential information relating to the respiratory muscles described above is a waveform image indicating a change in potential formed by synthesizing a change in the potential of the inspiratory muscles and a change in the potential of the expiratory muscles. Meanwhile, the input 2 may be a waveform image indicating only a change in the potential of the expiratory muscles or only a change in the potential of the inspiratory muscles. Alternatively, an image obtained by synthesizing the input 1 and the input 2 may be input to the exacerbation detection model 173. Alternatively, the input 1 may be time-series data of the information on the motion of the respiratory muscles, and the input 2 may be time-series data of the action potential information relating to the respiratory muscles. **In** addition, the input 1 and the input 2 may be a combination of a waveform image and time-series data.

The server 1 performs learning using training data in which an image of an electrical signal relating to the motion information and an image of the action potential information are associated with information relating to exacerbation in the images. The training data is data in which an image of an electrical signal relating to the motion information, an image of the action potential information, and information relating to exacerbation are labeled. The server 1 inputs a waveform image which is training data to the input layer, and acquires an identification result indicating the presence or absence of exacerbation in COPD from the output layer through an arithmetic operation process in the intermediate layer. Meanwhile, the identification result which is output from the output layer may be a value discretely indicating the presence or absence of exacerbation (for example, a value of "0" or "1"), or may be a continuous probability value (for example, a value in the range of "0" to "1"). For example, as the identification result which is output from the output layer, the server 1 acquires an identification result of identifying the probability value of exacerbation or the like in addition to the presence or absence of exacerbation in COPD. Alternatively, probability values of a plurality of levels, for example, level 1 to level 4, may be output from the output layer.

The server 1 compares the identification result output from the output layer with information labeled for a waveform image in the training data, that is, a correct answer value, and optimizes parameters used for the arithmetic operation process in the intermediate layer so that the output value from the output layer approaches the correct answer value. The parameters are, for example, a weight (coupling coefficient) between neurons, a coefficient of an activation function used in each neuron, and the like. A method of optimizing the parameters is not particularly limited, and, for example, the server 1 optimizes various parameters using a reverse error propagation method.

The server 1 performs the above process on each waveform image included in the training data, and generates the exacerbation detection model 173. In a case where the image of an electrical signal relating to the motion information is acquired from the detection sensor 3 and the image of action potential information is acquired from the electromyogram sensor 4, the server 1 detects the information relating to exacerbation in COPD in accordance with a command from the learned exacerbation detection model 173 stored in a memory. Meanwhile, in a case where the server 1 generates the learned exacerbation detection model 173 and then the terminal 2 downloads and installs the learned exacerbation detection model 173, the terminal 2 may detect the information relating to exacerbation using the learned exacerbation detection model 173. In this case, the terminal 2 acquires an image of an electrical signal relating to the motion information from the detection sensor 3 and an image of the action potential information from the electromyogram sensor 4, and detects the information relating to exacerbation using the exacerbation detection model 173. Meanwhile, the terminal 2 may perform a learning or re-learning process on the exacerbation detection model 173 using the training data.

Meanwhile, although the waveform image is used as the input value of the exacerbation detection model 173 in the present embodiment, there is no limitation thereto. For example, a neural network related to a recurrent neural network (RNN) may be used as the exacerbation detection model 173. In this case, the information relating to exacerbation in COPD may be detected using the motion information acquired from the detection sensor 3 and the time-series data of the action potential information acquired from the electromyogram sensor 4 as input values.

In addition, as the learning model, two models, that is, a first learning model learned on the basis of training data including the motion information and the information relating to exacerbation and a second learning model learned on the basis of training data including the action potential information and the information relating to exacerbation, may be prepared. In this case, in the first learning model, the voltage waveform shown in Fig. 8, a voltage waveform image, a frequency waveform obtained by frequency-converting the voltage waveform, or an image of the frequency waveform is used as input data, and the probability of exacerbation is output data. The server 1 learns the first learning model on the basis of the training data.

In the second learning model, the action potential waveforms related to the inspiratory muscles and the expiratory muscles shown in Figs. 10A and 10B, an action potential waveform image, a frequency waveform obtained by frequency-converting the action potential waveform, or an image of the frequency waveform is used as input data, and the probability of exacerbation is output data. The server 1 learns the second learning model on the basis of the training data. Meanwhile, the second learning model may be learned on the basis of only the waveform image related to the expiratory muscles or the inspiratory muscles.

In a case where the output probability of the first learning model during estimation is equal to or greater than a predetermined threshold (for example, 80%), or in the case of YES in step S205, an estimation process based on the second learning model is performed. The control unit 21 inputs an action potential waveform related to the inspiratory muscles and the expiratory muscles, an action potential waveform image, a frequency waveform obtained by frequency-converting the action potential waveform, or an image of the frequency waveform into the second learning model. The control unit 21 outputs the probability of exacerbation from the second learning model.

Meanwhile, there is no limitation to the order of the estimation processes based on the first learning model and the second learning model described above. For example, in a case where the estimation process based on the second learning model is performed and the probability of exacerbation output from the second learning model is equal to or higher than a predetermined threshold, the estimation process based on the first learning model may be performed. Alternatively, the estimation process based on the first learning model and the estimation process based on the second learning model may be performed in parallel, and exacerbation may be determined on the basis of the probability of exacerbation output using the first learning model and the second learning model. In addition, the first condition may be determined using the method of Embodiment 1, and the second learning model may be used for the action potential waveform. Further, the first learning model may be used for the first condition, and the action potential waveform may be determined using the method of Embodiment 1.

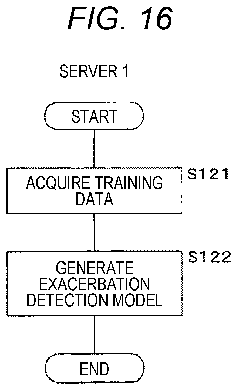

Fig. 16 is a flowchart illustrating an example of a processing procedure of a process of generating the exacerbation detection model 173. The process content of a process of generating the exacerbation detection model 173 will be described with reference to Fig. 16.

The control unit 11 acquires training data in which the image of the motion information acquired by the detection sensor 3 and the image of the action potential information acquired by the electromyogram sensor 4 are associated with the information relating to exacerbation in COPD (step S121). The training data is, for example, data in which the images of the motion information and the action potential information and the information relating to exacerbation (such as, for example, the probability value of exacerbation) are labeled.

The control unit 11 uses a plurality of pieces of training data to generate the exacerbation detection model 173 (learned model) that outputs information (for example, level 1 to level 4) indicating the presence or absence of the information relating to exacerbation in COPD in a case where the waveform images of the motion information and the action potential information are input (step S122). Specifically, the control unit 11 inputs the waveform image which is training data into the input layer of the neural network, and acquires the identification result of identifying the information relating to exacerbation from the output layer. The control unit 11 compares the acquired identification result with the correct answer value of the training data (information labeled for the waveform image), and optimizes parameters (such as a weight) used for the arithmetic operation process in the intermediate layer so that the identification result which is output from the output layer approaches the correct answer value. The control unit 11 stores the generated exacerbation detection model 173 in the high-capacity storage unit 17, and ends the series of processes.

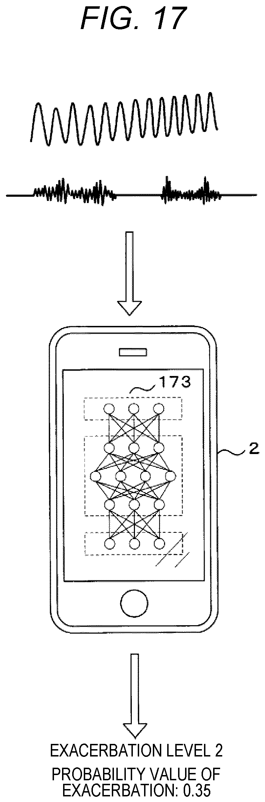

Fig. 17 is a diagram illustrating a process of detecting information relating to exacerbation using the exacerbation detection model 173. Fig. 17 conceptually illustrates a state in which the information relating to exacerbation in COPD is detected from the waveform image. The process of detecting the information relating to exacerbation in COPD will be described with reference to Fig. 17.

The control unit 21 of the terminal 2 in which the learned exacerbation detection model 173 is installed acquires an image of an electrical signal relating to the motion information from the detection sensor 3 and an image of the action potential information from the electromyogram sensor 4 through the Bluetooth communication unit 26. Meanwhile, the motion information and the action potential information are not limited to the above-described waveform image, and may be time-series data. In a case where the motion information and the action potential information are time-series data, the control unit 21 may generate a waveform image on the basis of the acquired time-series data. The control unit 21 inputs the acquired image of an electrical signal relating to the motion information and the acquired image of the action potential information into the exacerbation detection model 173.

The control unit 21 performs an arithmetic operation process of extracting the feature amount of the waveform image in the intermediate layer of the exacerbation detection model 173, inputs the extracted feature amount into the output layer of the exacerbation detection model 173, and acquires information relating to the presence or absence of exacerbation in COPD as an output. As shown in the drawing, the control unit 21 outputs the probability value of exacerbation and information on exacerbation levels (for example, exacerbation level 1 to exacerbation level 4) classified in accordance with the probability value of exacerbation. In a case where the information relating to exacerbation is detected from the waveform image using the exacerbation detection model 173, the control unit 21 displays the detection result indicating that the information relating to exacerbation in COPD has been detected on the display unit 25.

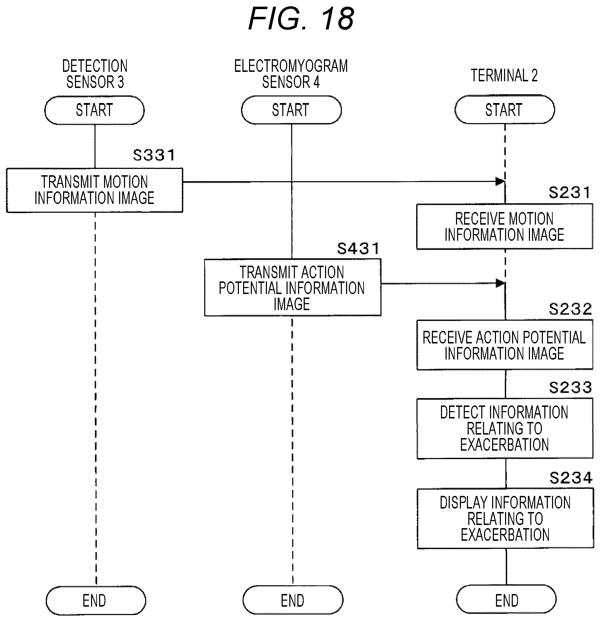

Fig. 18 is a flowchart illustrating a processing procedure of detecting the information relating to exacerbation using the exacerbation detection model 173. The control unit 31 of the detection sensor 3 transmits the image of an electrical signal relating to the motion information to the terminal 2 through the Bluetooth communication unit 34 (step S331). The control unit 21 of the terminal 2 in which the learned exacerbation detection model 173 is installed receives the image of an electrical signal relating to the motion information transmitted from the detection sensor 3 through the Bluetooth communication unit 26 (step S231).

Meanwhile, the control unit 21 may receive raw data of an electrical signal related to the motion information from the detection sensor 3 through the Bluetooth communication unit 26. In this case, for example, the control unit 21 may input the received raw data of the electrical signal into a deep-learned image generation model, and output an image of an electrical signal using the image generation model.

The control unit 41 of the electromyogram sensor 4 transmits the image of the action potential information to the terminal 2 through the Bluetooth communication unit 44 (step S431). The control unit 21 of the terminal 2 receives the image of the action potential information transmitted from the electromyogram sensor 4 through the Bluetooth communication unit 26 (step S232). The control unit 21 detects the information relating to exacerbation in COPD using the exacerbation detection model 173 (step S233), and displays the detected information relating to exacerbation on the display unit 25 (step S234).

According to the present embodiment, it is possible to make a quick and accurate diagnosis by detecting an abnormality in a respiratory system disease using the exacerbation detection model 173.

### (Embodiment 3)

Embodiment 3 relates to a mode of detecting an abnormality in a respiratory system disease by further adding biological information of a patient to motion information relating to the motion of the respiratory muscles or the accessory respiratory muscles and action potential information relating to the respiratory muscles or the accessory respiratory muscles. Meanwhile, in Embodiment 3, description will be given with an example of detecting information relating to exacerbation in COPD which is one respiratory system disease.

In the present embodiment, a second sensor 5 that detects biological information of a patient is included. The second sensor 5 includes, for example, an oximeter that detects percutaneous-arterial blood oxygen saturation, a microphone that collects biological sounds emitted from a living body, an electrocardiograph capable of recording the action potential or action current of the cardiac muscles associated with a heartbeat and thereby analyzing RRI (R-R Interval) and CV-RR (Coefficient of Variation R-R interval), a sphygmomanometer that detects blood pressure, a thermometer that detects body temperature, a measurement device of percutaneous-arterial blood carbon dioxide partial pressure (PtcCO2), a pulsimeter that measures a heartbeat rate, a heart rate variability meter that analyzes the frequency of a heartbeat, a bioelectric potential sensor that measures bioelectric impedance, a perspiration sensor, or the like.

The bioelectric potential sensor detects a change in bioelectric impedance associated with respiration. The change in bioelectric impedance involves ascertaining the ventilation condition by measuring thoracic impedance (Z) obtained by applying a high frequency current from electrodes arranged on the thorax and using respiratory impedance (ΔZ) which is the amount of change in the thoracic impedance according to a change in lung volume associated with ventilation. That is, the amount of change (ΔZ) in the thoracic impedance (Z) associated with respiration changes in accordance with the amount of change (ΔV_{L}) in the amount of air in the lungs (V_{L}). The relation of ΔZ = α (non-negative proportional coefficient)·ΔV_{L} can be assumed. The amount of change ΔZ in thoracic impedance increases with inspiration (ΔZ > 0), and decreases with expiration (ΔZ < 0). Therefore, the amount of ventilation of the lungs can be estimated by measuring the bioelectric potential impedance.

In addition, the second sensor 5 may be a non-contact type vital sensor installed under a bed, bedclothes, or the like without touching a human body. The non-contact type vital sensor uses a small-sized microwave radar to measure minute movements (heartbeats or breaths) on the body surface associated with respiratory movement and the beating of the heart, and uses an infrared thermo-camera to measure infrared rays (body temperature) emitted from the body surface. The biological information is a signal reflecting the state of a biological tissue and information in a case where the tissue is functioning, and is, for example, a signal emitted from the interior of the body due to biological phenomena such as pulse, body movement, or respiration.

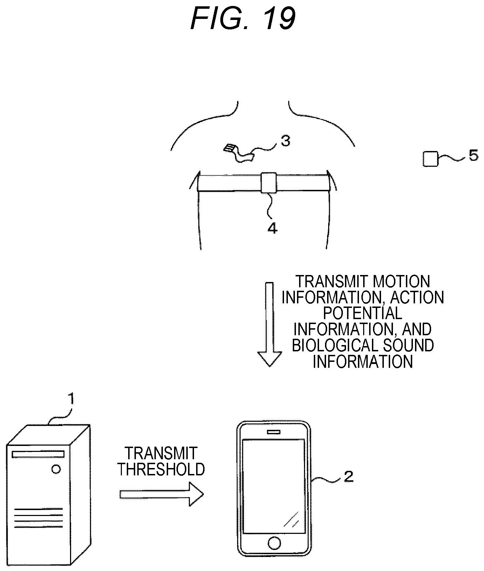

Fig. 19 is an explanatory diagram illustrating an operation of a process of detecting information relating to exacerbation by adding the second sensor 5. Meanwhile, in Fig. 19, description will be given with an example of detecting information relating to exacerbation in COPD using a microphone which is one type of second sensor 5. Meanwhile, there is no limitation to a microphone, and the information relating to exacerbation may be detected using, for example, a plurality of second sensors 5. A microphone is a sound sensor that collects biological sounds emitted from a living body. For example, using a microphone, it is possible to acquire biological sound information including a respiratory sound, a cough sound, a sputum retention sound, a pulmonary sound, or the like and to perform frequency analysis as well, and the information is input into a learning model.

In a case where the percutaneous-arterial blood carbon dioxide partial pressure (PtcCO2) measurement device is used, a value corresponding to PtcCO2 is input into the learning model. During exacerbation, PtcCO2 tends to increase. In addition, in a case where PtcCO2 becomes extremely high, the depression of respiration is observed, and fluctuation also occurs in the motion information and the action potential information. When learning the learning model, the learning process is performed using the training data including the motion information, the action potential information, and a value corresponding to PtcCO2 obtained on the basis of a history (measurement history of a patient in the past) and the exacerbation level.

In a case where the electrocardiogram is used, a potential which is output from the electrocardiograph is input into the learning model. During exacerbation, right heart strain findings are likely to be observed on the electrocardiogram. In addition, during exacerbation, arrhythmia called atrial fibrillation is likely to occur, and a corresponding change in potential occurs. Besides, during exacerbation, the sympathetic nerve becomes dominant over the parasympathetic nerve due to a poor physical condition, and accordingly, the shortening of RRI (interval between an R wave and an R wave on the electrocardiogram) and a decrease in CV-RR which is a fluctuation coefficient of RRI are observed. When learning the learning model, the learning process is performed using the training data including the motion information, the action potential information, and the output potential of the electrocardiograph obtained on the basis of the history and the exacerbation level.

In a case where the sphygmomanometer is used, blood pressure is input into the learning model. During exacerbation, the sympathetic nerve becomes dominant as described above, and in this case, the blood pressure rises. When learning the learning model, the learning process is performed using the training data including the motion information, the action potential information, and the blood pressure obtained on the basis of the history and the exacerbation level.

In a case where the thermometer is used, body temperature obtained from the thermometer is input into the learning model. The exacerbation may be triggered by respiratory tract infections caused by bacteria or viruses, and in this case, an increase in body temperature is observed as an inflammatory finding. When learning the learning model, the learning process is performed using the training data including the motion information, the action potential information, and the body temperature obtained on the basis of the history and the exacerbation level.

In a case where the pulsimeter is used, a pulse rate per unit time or a temporal change in the pulse rate is input into the learning model. During exacerbation, the sympathetic nerve becomes dominant as described above, and as a result, an increase in the pulse rate is observed. When learning the learning model, the learning process is performed using the training data including the motion information, the action potential information, and the pulse rate obtained on the basis of the history and the exacerbation level.

In a case where the heart rate variability meter is used, a ratio of low frequency (LF) / high frequency (HF) per unit time or a temporal change in the ratio of LF/HF is input into the learning model. During exacerbation, the sympathetic nerve becomes dominant as described above, and as a result, an increase in the ratio of LF/HF is observed. When learning the learning model, the learning process is performed using the training data including the motion information, the action potential information and, the ratio of LF/HF obtained on the basis of the history and the exacerbation level.

In a case where the bioelectric potential sensor is used, the amount of change ΔZ in the thoracic impedance per unit time or a temporal change in the amount of change ΔZ in the thoracic impedance is input into the learning model. During exacerbation, a decrease in a lung volume change associated with ventilatory impairment is observed, and as a result, a decrease in the amount of change ΔZ in the thoracic impedance is observed. When learning the learning model, the learning process is performed using the training data including the motion information, the action potential information, and the amount of change ΔZ in the thoracic impedance obtained on the basis of the history and the exacerbation level.

In a case where the perspiration sensor is used, the amount of sweating per unit time or a temporal change in the amount of sweating is input into the learning model. During exacerbation, the sympathetic nerve becomes dominant as described above, and as a result, an increase in the amount of sweating is observed. When learning the learning model, the learning process is performed using the training data including the motion information, the action potential information, and the amount of sweating obtained on the basis of the history and the exacerbation level. Meanwhile, a plurality of second sensors described above may be combined and input into the learning model.

The control unit 21 of the terminal 2 acquires the motion information relating to the motion of the respiratory muscles or the accessory respiratory muscles from the detection sensor 3 and the action potential information relating to the respiratory muscles or the accessory respiratory muscles from the electromyogram sensor 4 through the Bluetooth communication unit 26. The control unit 21 acquires biological sound information from a microphone through the Bluetooth communication unit 26. For example, biological sound information such as frequency, intensity, duration, or sound quality may be acquired with respect to a respiratory sound, a cough sound, a sputum retention sound, or a pulmonary sound. The control unit 21 detects the information relating to exacerbation in COPD on the basis of the acquired motion information, action potential information, and biological sound information.

For example, based on the process of detecting the information relating to exacerbation on the basis of the motion information and the action potential information described in Embodiment 1, the biological sound information may be further added to detect the information relating to exacerbation. Specifically, in a case where the information relating to exacerbation is detected on the basis of the motion information and the action potential information, the control unit 21 of the terminal 2 continues to determine exacerbation on the basis of the biological sound information (such as, for example, a respiratory sound) acquired by a microphone. In a case where exacerbation occurs in a COPD patient, biological reactions such as the attenuation of a respiratory sound, the prolongation of expiration, an increase in the number of coughs or sputa, an increase in a pulmonary sound, or the like occurs, and thus the control unit 21 detects the information relating to exacerbation on the basis of the biological sound information. The control unit 21 displays the detected information relating to exacerbation on the display unit 25. The control unit 21 transmits the detected information relating to exacerbation to the server 1 through the communication unit 23. The control unit 11 of the server 1 receives the information relating to exacerbation transmitted from the terminal 2 through the communication unit 13, and stores the received information relating to exacerbation in the patient DB 171 of the high-capacity storage unit 17.

Meanwhile, although the second sensor 5, the detection sensor 3, and the electromyogram sensor 4 are shown separately in the drawing of the present embodiment, they may be configured integrally. In addition, the location of the second sensor 5 is not limited to a specific region of the body, and may be a non-contact type. In addition, although the terminal 2 and the second sensor 5 are shown separately in the drawing of the present embodiment, there is no limitation thereto. For example, a device configuration may be such that the terminal 2, the detection sensor 3, the electromyogram sensor 4, and the second sensor 5 are all integrated, or a device configuration in which the terminal 2 and the second sensor 5 are integrated (for example, a wearable device such as a wristwatch-type mobile terminal). In addition, in a case where the second sensor 5 having a watch shape is worn by a user and an abnormality is detected by the second sensor 5, the detection sensor 3 and the electromyogram sensor 4 may be attached to the user's body.

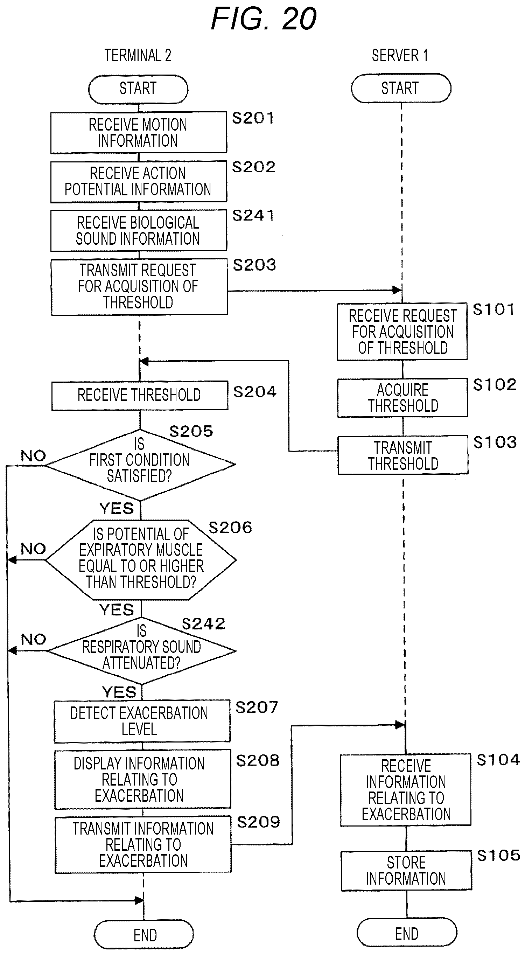

Fig. 20 is a flowchart illustrating a processing procedure of detecting information relating to exacerbation by adding a microphone. Meanwhile, content overlapping that of Fig. 12 is denoted by the same reference numerals and signs and thus description thereof will be omitted. The control unit 21 of the terminal 2 acquires biological sound information (such as, for example, a respiratory sound) from a microphone through the Bluetooth communication unit 26 (step S241). In a case where the information relating to exacerbation in COPD is detected on the basis of the motion information and the action potential information (steps 203 to 206), the control unit 21 determines whether the respiratory sound is attenuated (step S242). For a process of determining attenuation of the respiratory sound, for example, determination may be made using the frequency of the respiratory sound.

In a case where it is determined that the acquired respiratory sound is not attenuated (NO in step S242), the control unit 21 ends the process. In a case where it is determined that the acquired respiratory sound is attenuated (YES in step S242), the control unit 21 executes step S207. Meanwhile, although an example of one respiratory sound which is biological sound information has been described in Fig. 20, there is no limitation thereto. The information relating to exacerbation in COPD may be detected by another cough sound, a sputum retention sound, a pulmonary sound, or a combination of various types of sound information described above.

Meanwhile, although an example in which the determination of whether the respiratory sound is attenuated (step S242) is performed after the exacerbation determination process (step S205) based on the motion information and the exacerbation determination process (step S206) based on the action potential information has been described in the above-described process, there is no limitation thereto. For example, the information relating to exacerbation may be detected by first performing the determination of whether the respiratory sound is attenuated (step S242), then performing the exacerbation determination process (step S206) based on the action potential information, and next performing the exacerbation determination process (step S205) based on the motion information.

Alternatively, in a case where the determination of whether the respiratory sound is attenuated (step S242), the exacerbation determination process (step S205) based on the motion information, and the exacerbation determination process (step S206) based on the action potential information are performed in parallel and the exacerbation determination condition derived using the three is satisfied, the information relating to exacerbation may be detected.

Next, a process of detecting information relating to exacerbation will be described using a learned model constructed by deep learning on the basis of motion information, action potential information, and biological information acquired by the second sensor 5.

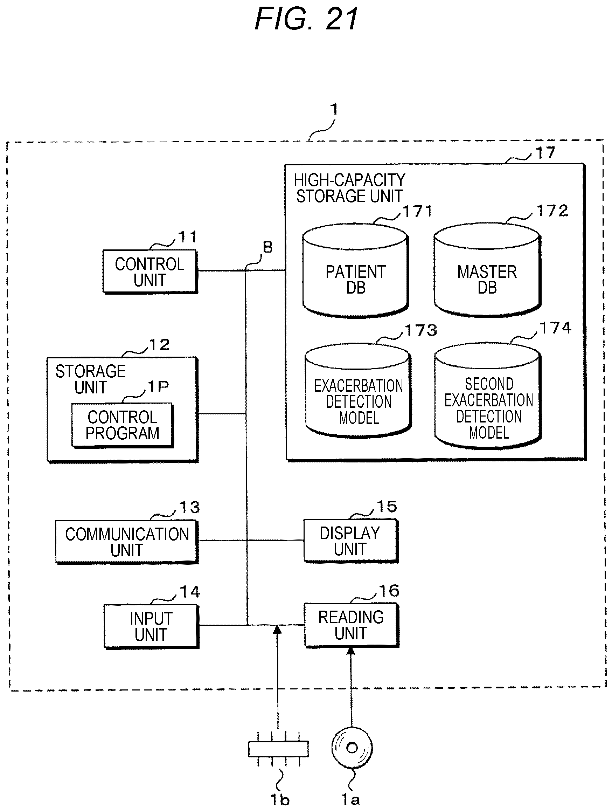

Fig. 21 is a block diagram illustrating a configuration example of a server 1 of Embodiment 3. Meanwhile, content overlapping that of Fig. 14 is denoted by the same reference numerals and signs and thus description thereof will be omitted. The high-capacity storage unit 17 includes a second exacerbation detection model 174. The second exacerbation detection model 174 is an exacerbation detection model constructed by deep learning. The second exacerbation detection model 174 constructs (generates) a neural network in which a waveform image is used as an input and information indicating the presence or absence of the information relating to exacerbation in COPD is used as an output by performing deep learning to learn the feature amount of the waveform image of an exacerbation point within an image of an electrical signal relating to motion information, an image of action potential information, and a biological signal image acquired by the second sensor 5. Meanwhile, a process of generating the second exacerbation detection model 174 is the same as the process of generating the exacerbation detection model 173 of Embodiment 1, and thus description thereof will not be given.

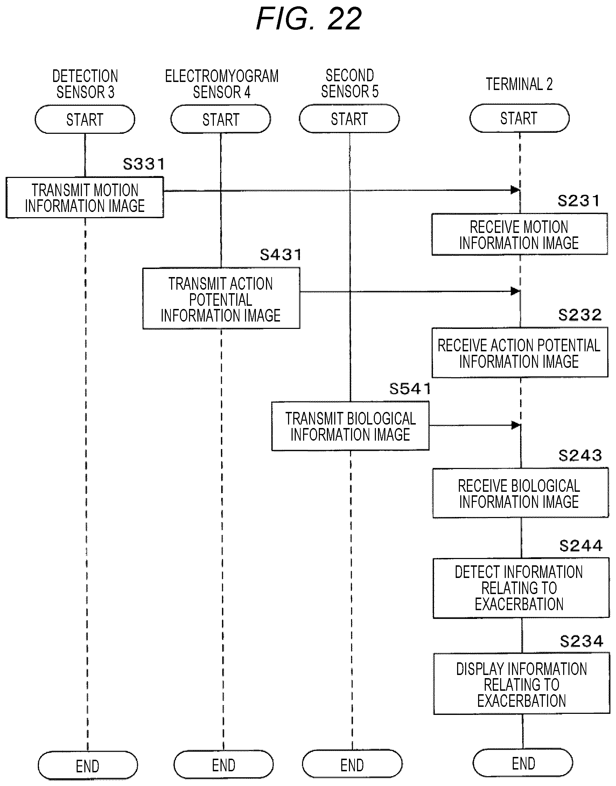

Fig. 22 is a flowchart illustrating a processing procedure of detecting information relating to exacerbation using the second exacerbation detection model 174. Meanwhile, content overlapping that of Fig. 18 is denoted by the same reference numerals and signs and thus description thereof will be omitted. The second sensor 5 transmits an image of biological information to the terminal 2 using Bluetooth communication (step S541). The control unit 21 of the terminal 2 receives the image of the biological information transmitted from the second sensor 5 through the Bluetooth communication unit 26 (step S243).

The biological information may be, for example, oxygen dissociation curve data acquired by an oximeter that detects percutaneous-arterial blood oxygen saturation. In a case where a COPD patient suffers from exacerbation, the percutaneous oxygen concentration decreases, and thus the information relating to exacerbation can be detected on the basis of an image of the oxygen dissociation curve data. Meanwhile, there is no limitation to the percutaneous-arterial blood oxygen saturation. The image of the biological information may be, for example, a flag indicating a temporal change in time-series data with respect to a respiratory rate or a heart rate, waveform data of a microphone, or the like.

The control unit 21 detects the information relating to exacerbation in COPD using the second exacerbation detection model 174 installed in the terminal 2 (step S244). Meanwhile, a process of detecting information relating to exacerbation using the second exacerbation detection model 174 is the same as the process of detecting information relating to exacerbation using the exacerbation detection model 173 of Embodiment 1, and thus description thereof will not be given.

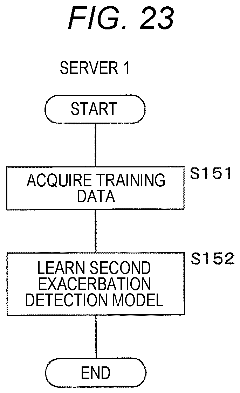

Fig. 23 is a flowchart illustrating an example of a processing procedure of a process of learning the second exacerbation detection model 174. The process content of a process of learning the second exacerbation detection model 174 will be described with reference to Fig. 23.

The control unit 11 acquires training data in which an image of motion information acquired by the detection sensor 3, an image of action potential information acquired by the electromyogram sensor 4, and an image of biological information acquired by the second sensor 5 are associated with the information relating to exacerbation in COPD (step S151). The training data is, for example, data in which the images of the motion information, the action potential information, and the biological information and the information relating to exacerbation (such as, for example, the probability value of exacerbation) are labeled.

The control unit 11 learns the second exacerbation detection model 174 that outputs information relating to the presence or absence of exacerbation in a case where the images of the motion information, the action potential information, and the biological information are input using the training data (step S152). Specifically, the control unit 11 inputs the waveform image which is training data into the input layer of the neural network, and acquires the identification result of identifying the information relating to exacerbation from the output layer. The control unit 11 compares the acquired identification result with the correct answer value of the training data (information labeled for the waveform image), and optimizes parameters (such as a weight) used for the arithmetic operation process in the intermediate layer so that the identification result which is output from the output layer approaches the correct answer value. The control unit 11 stores the learned second exacerbation detection model 174 in the high-capacity storage unit 17, and ends the series of processes.

According to the present embodiment, an abnormality in a respiratory system disease can be detected on the basis of the motion information acquired by the detection sensor 3, the action potential information acquired by the electromyogram sensor 4, and the biological information acquired by the second sensor 5.

According to the present embodiment, using the biological information as an auxiliary means, the information relating to exacerbation is detected together with the motion information and the action potential information. Thereby, for example, even when the information relating to exacerbation is erroneously detected by the detection process of Embodiment 1, the information relating to exacerbation can be corrected on the basis of the biological information.

### (Embodiment 4)

Embodiment 4 relates to a mode of detecting an abnormality in a respiratory system disease using a third sensor that further detects activity (exercise) information on the basis of Embodiment 3. Meanwhile, in Embodiment 4, description will be given with an example of detecting information relating to exacerbation in COPD which is one respiratory system disease. In the present embodiment, a third sensor 6 is included. The third sensor 6 detects activity information including the exercise amount, movement distance, activity amount, or posture of a patient, and is, for example, an acceleration sensor, a Global Positioning System/Global Positioning Satellite (GPS) sensor, a gyro sensor (angular velocity sensor), or the like. Meanwhile, the activity (exercise) information may be detected using a plurality of third sensors 6.

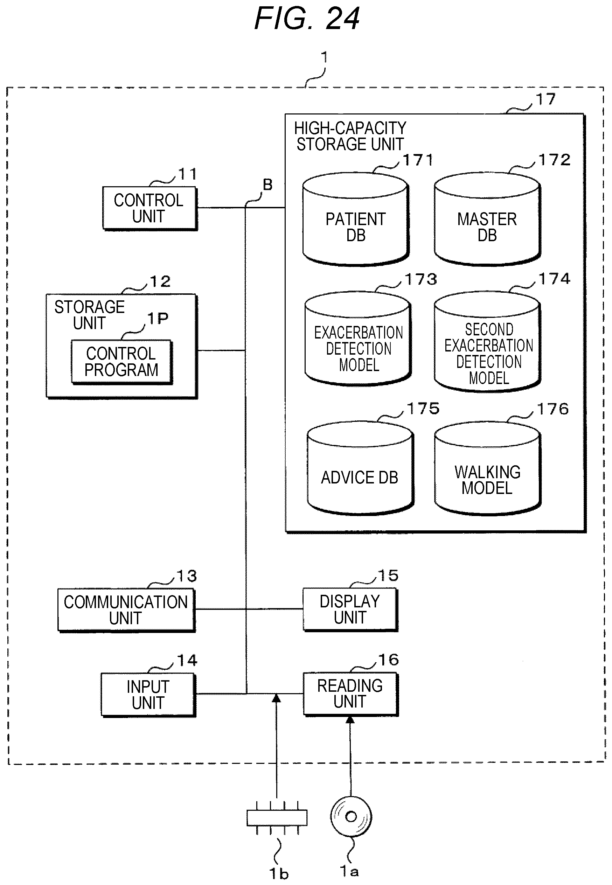

Fig. 24 is a block diagram illustrating a configuration example of a server 1 of Embodiment 4. Meanwhile, content overlapping that of Fig. 21 is denoted by the same reference numerals and signs and thus description thereof will be omitted. The high-capacity storage unit 17 includes an advice DB 175 and a walking model 176. The advice DB 175 stores advice information according to activity content, information relating to exacerbation, and the like.

The activity content includes, for example, walking, biking, stair climbing, jogging, respiratory rehabilitation exercise, and the like, and are acquired by the third sensor 6. For example, the control unit 21 of the terminal 2 monitors physical activity using an acceleration sensor, acquires acceleration data (activity information) such as posture, speed, or movement distance, and specifies the activity content in accordance with the acquired acceleration data. Meanwhile, the specification of the activity content is not limited to the above-described specific process. For example, in a case where a screen where the activity content can be selected is displayed to a user, the activity content may be specified by the user's touch operation.

The walking model 176 is a dedicated exacerbation detection model constructed by deep learning in a case where the activity content is walking. For example, the image of an electrical signal relating to the motion information, the image of the action potential information, and the biological signal image acquired by the second sensor 5 are input into the walking model 176. The walking model 176 constructs (generates) a neural network in which a waveform image is used as an input and information indicating the presence or absence of the information relating to exacerbation in COPD is used as an output by performing deep learning to learn the feature amount of the waveform image of an exacerbation point within the input image in accordance with walking. Meanwhile, a process of generating the walking model 176 is the same as the process of generating the exacerbation detection model 173 of Embodiment 1, and thus description thereof will not be given.

In addition, the walking model 176 may be generated on the basis of the motion information and the action potential information without using the biological signal image of the second sensor 5.

Meanwhile, in the present embodiment, description will be given with an example of the walking model 176, and each model (such as, for example, a bicycle model, a stair climbing model, a jogging model, or a respiratory rehabilitation model) may be used for each piece of activity content. For example, in a case where a respiratory rehabilitation activity is specified, the information relating to exacerbation may be detected using a learned respiratory rehabilitation model in which the image of an electrical signal relating to the motion information, the image of the action potential information, and the biological signal image acquired by the second sensor 5 are used as an input and the information relating to exacerbation in COPD is used as output. The respiratory rehabilitation model detects the information relating to exacerbation in accordance with the physical reaction status during respiratory rehabilitation by learning the feature amounts of the image of the motion information during respiratory rehabilitation detected by the detection sensor 3, the image of the action potential information during respiratory rehabilitation detected by the electromyogram sensor 4, and the biological signal image acquired by the second sensor 5.

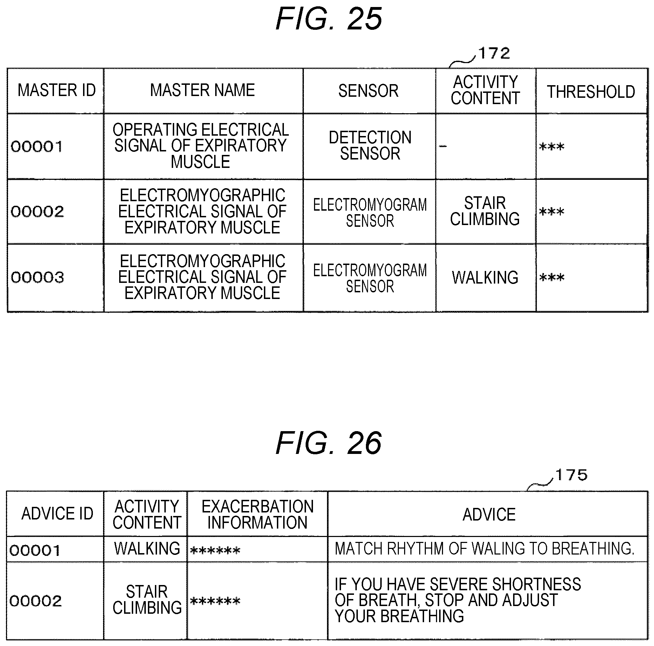

Fig. 25 is a diagram illustrating an example of a record layout of a master DB 172 of Embodiment 3. Meanwhile, content overlapping that of Fig. 4 is denoted by the same reference numerals and signs and thus description thereof will be omitted. The master DB 172 includes an activity content column. The activity content column stores the type of action in accordance with physical activity. For example, the activity content is stair climbing, walking, and the like.

Fig. 26 is a diagram illustrating an example of a record layout of the advice DB 175. The advice DB 175 includes an advice ID column, an activity content column, an exacerbation information column, and an advice column. The advice ID column stores the ID of advice which is uniquely specified in order to identify the advice. The activity content column stores the type of action in accordance with physical activity. The exacerbation information column stores information relating to exacerbation. The advice column stores advice information for a patient in accordance with information relating to the activity content and exacerbation.

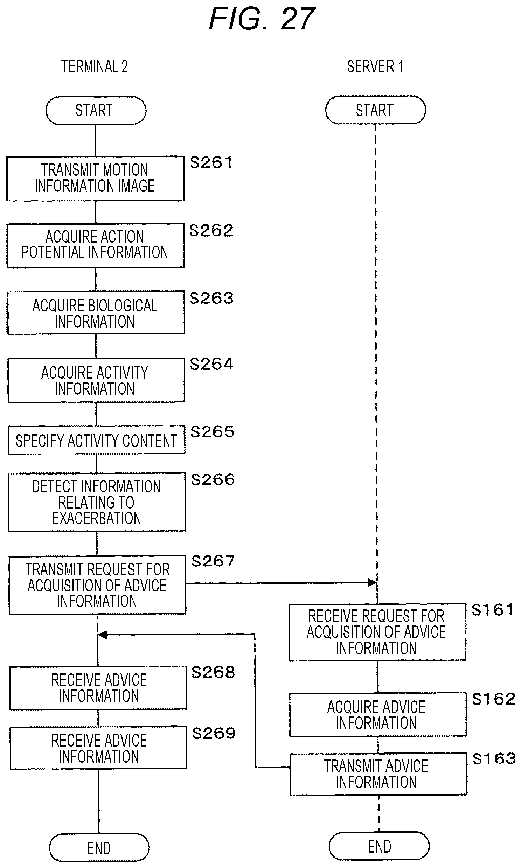

Next, a process of transmitting advice information according to the activity content will be described. Fig. 27 is a flowchart illustrating a processing procedure of transmitting advice information according to the activity content. The control unit 21 of the terminal 2 acquires motion information relating to the motion of the respiratory muscles or the accessory respiratory muscles from the detection sensor 3 through the Bluetooth communication unit 26 (step S261), and acquires action potential information relating to the respiratory muscles or the accessory respiratory muscles from the electromyogram sensor 4 (step S262). The control unit 21 acquires biological information using the second sensor 5 through the Bluetooth communication unit 26 (step S263), and acquires activity information including the exercise amount, movement distance, activity amount, or posture of a patient using the third sensor 6 (step S264).

The control unit 21 specifies the activity content on the basis of the acquired activity information (step S265). The control unit 21 detects the information relating to exacerbation on the basis of the acquired motion information, action potential information, and biological information (step S266). Meanwhile, a process of detecting the information relating to exacerbation is the same as the detection process in Embodiment 3, and thus description thereof will not be given. The control unit 21 transmits a request for the acquisition of advice to the server 1 through the communication unit 23 on the basis of the detected information relating to exacerbation and the specified activity content (step S267). The control unit 11 of the server 1 receives the request for the acquisition of advice transmitted from the terminal 2 through the communication unit 13 (step S161).

The control unit 11 acquires advice information from the advice DB 175 of the high-capacity storage unit 17 in accordance with the received request for the acquisition of advice (step S162). The control unit 11 transmits the acquire advice information to the terminal 2 through the communication unit 13 (step S163). The control unit 21 of the terminal 2 receives the advice information transmitted from the server 1 through the communication unit 23 (step S268), and displays the received advice information on the display unit 25 (step S269). For example, the advice information "Match rhythm of running to breathing!" may be displayed to a patient who is jogging with the exacerbation level 2 (low risk). In addition, the advice information "Stop and adjust your breathing!" may be displayed to a patient who is climbing stairs with the exacerbation level 3 (high risk). In addition, the advice information "Please use short-acting inhaled β2 stimulant!" may be displayed to a patient who is walking with the exacerbation level 4 (exacerbation).

Next, a process of detecting information relating to exacerbation in COPD using a model for each activity constructed by machine learning will be described. Meanwhile, in the present embodiment, description will be given on the basis of the walking model 176 that can be used when walking, but the same applies to other activity content. Since the exercise amount, activity amount, posture, and the like differ depending on the activity content, which leads to influencing the detection result of detecting the information relating to exacerbation in COPD. It is possible to construct a neural network in which waveform images are used as an input and information indicating the presence or absence of the information relating to exacerbation in COPD is used as an output by performing deep learning to learn the feature amounts of various waveform images acquired from the state of a specific activity (for example, walking). Thus, using the learned dedicated model leads to an increase in the accuracy of detecting the information relating to exacerbation in COPD in the specific activity.

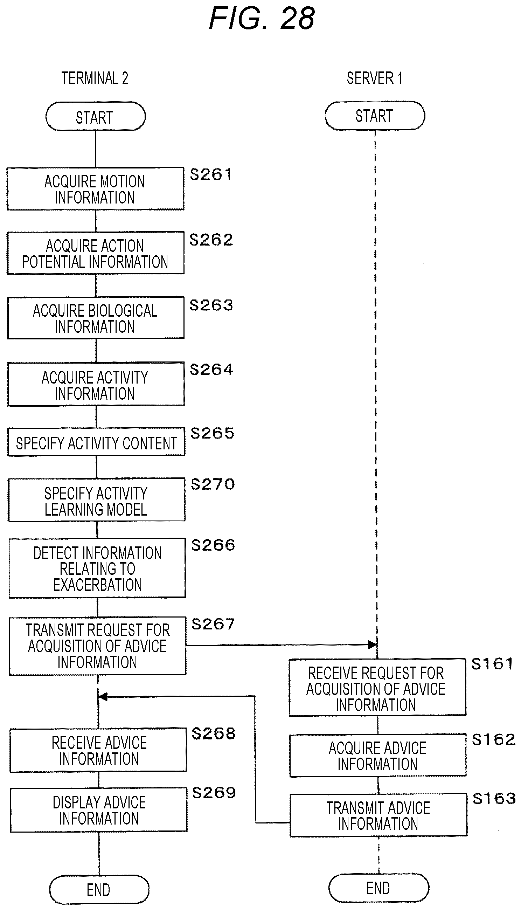

Fig. 28 is a flowchart illustrating a processing procedure of detecting information relating to exacerbation using the walking model 176. Meanwhile, content overlapping that of Fig. 27 is denoted by the same reference numerals and signs and thus description thereof will be omitted. The control unit 21 of the terminal 2 specifies a learned activity learning model in accordance with the activity content specified in step S265 (step S270). For example, in a case where the activity content of walking is specified from the activity information acquired in step S264, the control unit 21 specifies the walking model 176 installed in advance, and detects the information relating to exacerbation in COPD. That is, by using a learned activity learning model for each piece of activity content, it is possible to detect high-accuracy information relating to exacerbation in COPD.

Next, a process of changing a predetermined threshold of an electrical signal for determining the action potential information acquired by the electromyogram sensor 4 will be described.

Since the exercise amount or the like changes according to the activity content, a criterion for determination of information relating to the presence or absence of exacerbation in COPD is changed accordingly. Hereinafter, description will be given with an example of changing a predetermined threshold of an electrical signal for determining the action potential information acquired by the electromyogram sensor 4 in accordance with the activity content. The control unit 21 of the terminal 2 acquires the activity information detected by the third sensor 6 through the Bluetooth communication unit 26. The control unit 21 specifies the activity content in accordance with the acquired activity information. The control unit 21 acquires a predetermined threshold of an electrical signal for determining the action potential information acquired by the electromyogram sensor 4 from the master DB 172 of the high-capacity storage unit 17 of the server 1 on the basis of the specified activity content. The control unit 21 performs a determination process when the information relating to exacerbation is detected on the basis of an acquired new threshold. For example, in a case where the control unit 21 of the terminal 2 specifies the activity content as walking, the control unit 21 acquires a predetermined threshold of an electrical signal for determining the action potential information corresponding to walking from the master DB 172 of the high-capacity storage unit 17 of the server 1. In the case of walking, since the amount of exercise increases, the threshold of the electrical signal for detecting the information relating to exacerbation becomes higher than a threshold at rest.

Meanwhile, although the above-described example of a process of changing the threshold of an electrical signal has been described, there is no limitation thereto. It is possible to change the first condition for determining the motion information acquired by the detection sensor 3 and the predetermined threshold of the biological information for determining the biological information acquired by the second sensor 5. For example, in a case where the process of detecting exacerbation is performed using the detection sensor 3, the terminal 2 changes the operating electrical signal threshold of the expiratory muscles in accordance with the activity content. For example, in a case where the terminal 2 specifies the activity content as walking, the exercise amount increase, and thus the maximum slope threshold or the minimum slope threshold of the voltage is changed to be high.

In addition, in a case where the terminal 2 performs the process of detecting exacerbation using the second sensor 5, various types of thresholds of the second sensor 5 may be changed in accordance with the activity content. For example, in a case where the second sensor 5 is a microphone, the control unit 21 of the terminal 2 acquires audio data. The control unit 21 frequency-converts the audio data, and determines whether the power of a specific frequency band exceeds a predetermined threshold. In a case where the predetermined threshold is exceeded, the control unit 21 determines that there is a possibility of exacerbation, and performs a determination using the detection sensor 3 and the electromyogram sensor 4. Here, the control unit 21 changes the above-described predetermined threshold in accordance with the activity content.

Meanwhile, there is no limitation to the process of changing the predetermined threshold according to the above-described activity content. For example, using the exacerbation detection model 173, the predetermined threshold of an electrical signal for determining the action potential information acquired by the electromyogram sensor 4 may be changed in accordance with the output probability value of exacerbation.

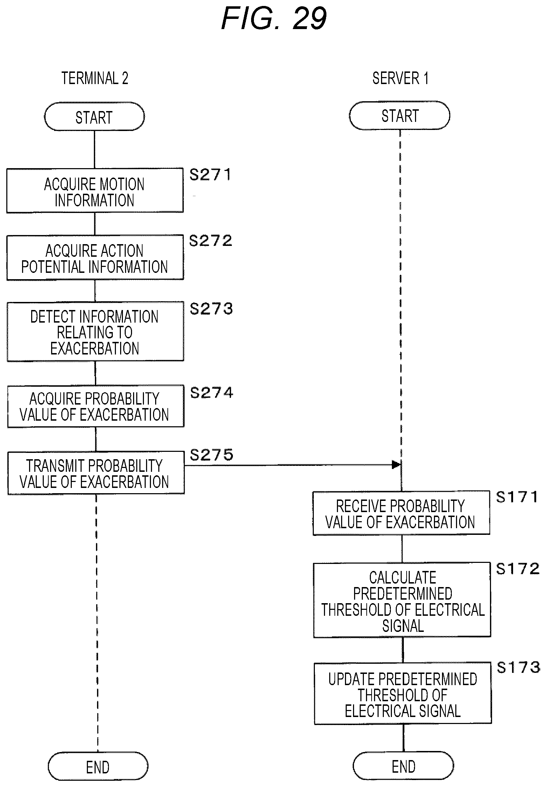

Fig. 29 is a flowchart illustrating processing procedure of changing a predetermined threshold of an electrical signal according to the probability value of exacerbation. The control unit 21 of the terminal 2 in which the learned exacerbation detection model 173 is installed acquires motion information relating to the motion of the respiratory muscles or the accessory respiratory muscles from the detection sensor 3 through the Bluetooth communication unit 26 (step S271). The control unit 21 acquires the action potential information relating to the respiratory muscles or the accessory respiratory muscles from the electromyogram sensor 4 through the Bluetooth communication unit 26 (step S272).

The control unit 21 detects the information relating to exacerbation in COPD using the exacerbation detection model 173 (step S273). The control unit 21 acquires the probability value of exacerbation from the detected information relating to exacerbation (step S274). For example, in a case where the acquired probability value of exacerbation is 0.3, there is a risk of developing exacerbation, and thus it is necessary to change a predetermined threshold of the electrical signal for determining the action potential information acquired by the electromyogram sensor 4.

The control unit 21 transmits the acquired probability value of exacerbation to the server 1 through the communication unit 23 (step S275). The control unit 11 of the server 1 receives the probability value of exacerbation transmitted from the terminal 2 through the communication unit 13 (step S 171). The control unit 11 calculates the predetermined threshold of the electrical signal for determining the action potential information acquired by the electromyogram sensor 4 in accordance with the received probability value of exacerbation (step S172). Regarding a calculation algorithm, for example, in a case where there is a risk of developing exacerbation, calculation may be performed by multiplying a predetermined threshold of an existing electrical signal by a coefficient between 0 and 1. That is, in a case where there is a risk of developing exacerbation, a criterion for determination of exacerbation detection becomes stricter. The control unit 11 updates the calculated predetermined threshold of the electrical signal to the master DB 172 of the high-capacity storage unit 17 (step S173).

Next, a process of detecting information relating to exacerbation in COPD using the detection sensor 3, the electromyogram sensor 4, and the third sensor 6 will be described. For example, the third sensor 6 is a gyro sensor. The terminal 2 uses the detection sensor 3 to acquire motion information relating to the motion of the respiratory muscles or the accessory respiratory muscles, uses the electromyogram sensor 4 to acquire action potential information relating to the respiratory muscles or the accessory respiratory muscles, and uses the gyro sensor to acquire a foot stagger value. The terminal 2 determines whether the acquired foot stagger value is equal to or higher than a predetermined threshold. In a case where it is determined that the acquire foot stagger value is equal to or higher than the predetermined threshold, the terminal 2 detects the information relating to exacerbation on the basis of the acquired motion information and action potential information through the same process as in Embodiment 1. Meanwhile, regarding the process of detecting the foot stagger, gyro sensor data acquired by the gyro sensor may be used as an input, and a learned stagger detection model that detects the stagger may be used.

Meanwhile, although the process of changing the predetermined threshold of the electrical signal for determining the action potential information acquired by the electromyogram sensor 4 has been described in the present embodiment, there is no limitation thereto. Other thresholds for determination may also be changed according to the above-described process content.

According to the present embodiment, regarding the detection of an abnormality in a respiratory system disease according to the activity content, it is also possible to increase the detection rate of exacerbations with respect to symptoms related to exacerbations which are difficult to find at rest.

According to the present embodiment, by using each learned activity learning model according to the activity content, it is possible to detect information relating to exacerbation in COPD even in the case of activities other than at rest (for example, high-intensity activities such as jogging) in consideration of the actual state of physical activity.

According to the present embodiment, it is possible to appropriately change a predetermined threshold of an electrical signal for determining information relating to the presence or absence of exacerbation in accordance with the activity content or the probability value of exacerbation.

### (Embodiment 5)

Embodiment 5 relates to a mode in which, in a case where information relating to exacerbation in COPD is detected, medication instruction information or second advice information other than the medication instruction information is transmitted in accordance with the detected information relating to exacerbation. Meanwhile, content overlapping that of Embodiments 1 to 4 will not be described.

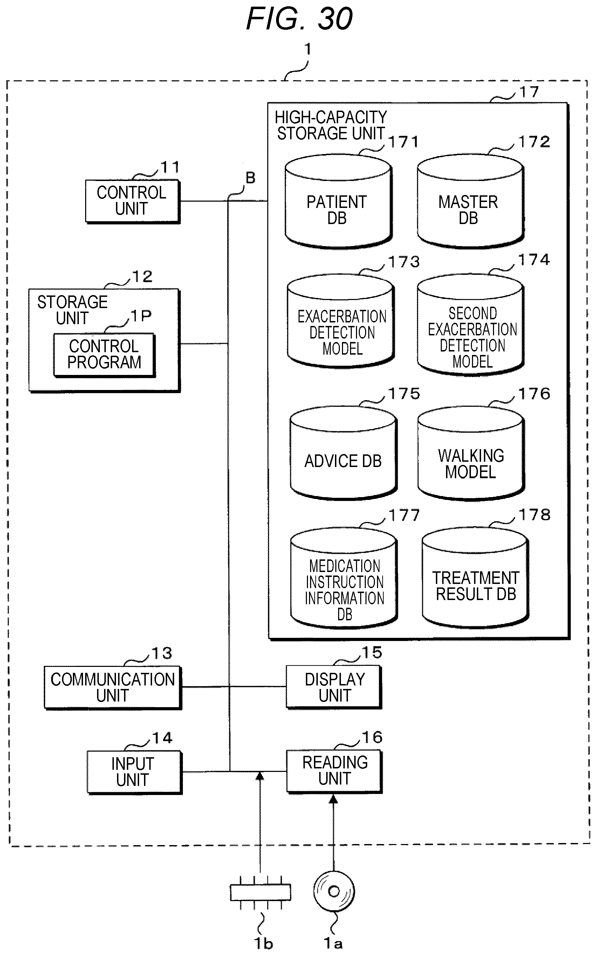

Fig. 30 is a block diagram illustrating a configuration example of a server 1 of Embodiment 5. Meanwhile, content overlapping that of Fig. 24 is denoted by the same reference numerals and signs and thus description thereof will be omitted. The high-capacity storage unit 17 includes a medication instruction information DB 177 and a treatment result DB 178. The medication instruction information DB 177 stores information on medication instructions for a medicine prescribed to be used at the time of exacerbation in advance for a COPD patient. The treatment result DB 178 stores information relating to the ingestion of a medicine prescribed to be used at the time of exacerbation in advance and measurement data after the medicine is ingested for a patient from whom information relating to exacerbation has been detected.

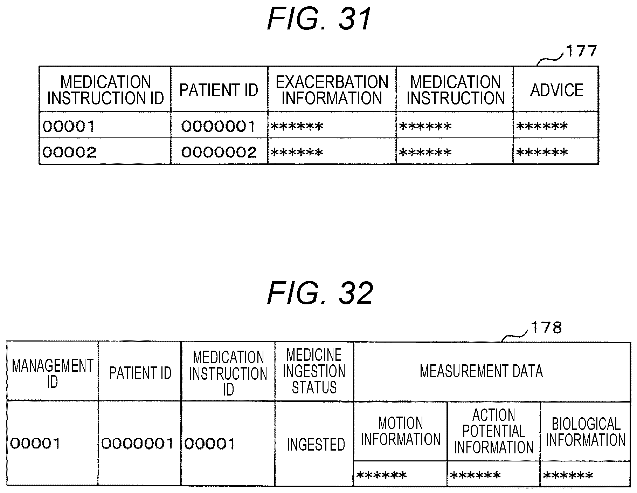

Fig. 31 is a diagram illustrating an example of a record layout of the medication instruction information DB 177. The medication instruction information DB 177 includes a medication instruction ID column, a patient ID column, an exacerbation information column, a medication instruction column, and an advice column. The medication instruction ID column stores the ID of the medication instruction which is uniquely specified in order to identify each medication instruction. The patient ID column stores the patient ID for specifying the patient. The exacerbation information column stores information relating to exacerbation. The medication instruction column stores information on medication instructions according to the information relating to exacerbation. The advice column stores the second advice information other than the information on medication instructions in accordance with the information relating to exacerbation.

Fig. 32 is a diagram illustrating an example of a record layout of the treatment result DB 178. The treatment result DB 178 includes a management ID column, a patient ID column, a medication instruction ID column, a medicine ingestion status column, and a measurement data column. The management ID column stores the ID of treatment result data which is uniquely specified in order to identify data of each treatment result. The patient ID column stores the patient ID for specifying the patient. The medication instruction ID column stores the medication instruction ID for specifying medication instructions. The medicine ingestion status column stores the status of ingestion of a medicine instructed to be taken by a patient from whom information relating to exacerbation has been detected. For example, "ingested," "ingesting," "not ingested," or the like may be entered in the medicine ingestion status column.

The measurement data column includes a motion information column, an action potential information column, and a biological information column. The motion information column stores motion information acquired by the detection sensor 3 with respect to a patient from whom information relating to exacerbation has been detected after the patient has taken a medicine instructed to take the medicine. The action potential information column stores action potential information acquired by the electromyogram sensor 4 with respect to a patient from whom information relating to exacerbation has been detected after the patient has taken a medicine instructed to take the medicine. The biological information column stores biological information acquired by the second sensor 5 with respect to a patient from whom information relating to exacerbation has been detected after the patient has taken a medicine instructed to take the medicine.

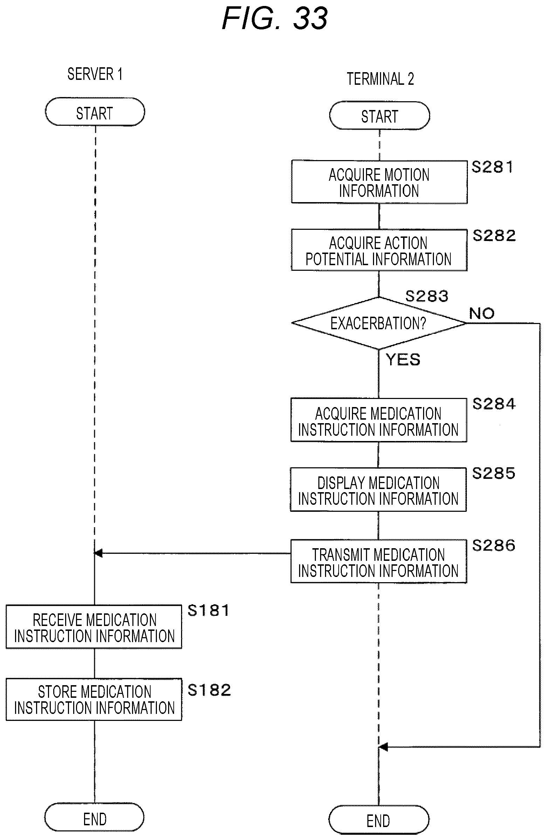

Fig. 33 is a flowchart illustrating a processing procedure of transmitting medication instruction information for a medicine prescribed to be used at the time of exacerbation in advance according to information relating to exacerbation. The control unit 21 of the terminal 2 acquires the motion information from the detection sensor 3 through the Bluetooth communication unit 26 (step S281), and acquires the action potential information from the electromyogram sensor 4 (step S282). The control unit 21 determines whether to detect the information relating to exacerbation in COPD (step S283). Meanwhile, a process of detecting the information relating to exacerbation is the same as the process of detecting the information relating to exacerbation in Embodiment 1, and thus description thereof will not be given. In a case where it is determined that the information relating to exacerbation in COPD is not detected (NO in step S283), the control unit 21 ends the process.

In a case where it is determined that the information relating to exacerbation in COPD is detected (YES in step S283), the control unit 21 acquires the medication instruction information according to the information relating to exacerbation (step S284). For example, the control unit 21 may accept information on direct medication instructions given to a medicine prescribed in advance by a doctor after confirming the information relating to exacerbation through the communication unit 23 or the input unit 24, or may acquire information on medication instructions for a medicine prescribed in advance from the storage unit 22 in accordance with the information relating to exacerbation. The medication instruction information includes the type of medicine (such as, for example, a short-acting inhaled β2 stimulant), a name (for example, meptin), a medication method, side effects, and the like. The control unit 21 displays the acquired medication instruction information on the display unit 25 (step S285). The control unit 21 transmits the acquired medication instruction information to the server 1 through the communication unit 23 (step S286).

The control unit 11 of the server 1 receives the medication instruction information according to the information relating to exacerbation transmitted from the terminal 2 through the communication unit 13 (step S181), and stores the received medication instruction information in the medication instruction information DB 177 of the high-capacity storage unit 17 (step S182). Specifically, the control unit 11 allocates a medication instruction ID, and stores the patient ID, the information relating to exacerbation, and the medication instruction information as one record in the medication instruction information DB 177.

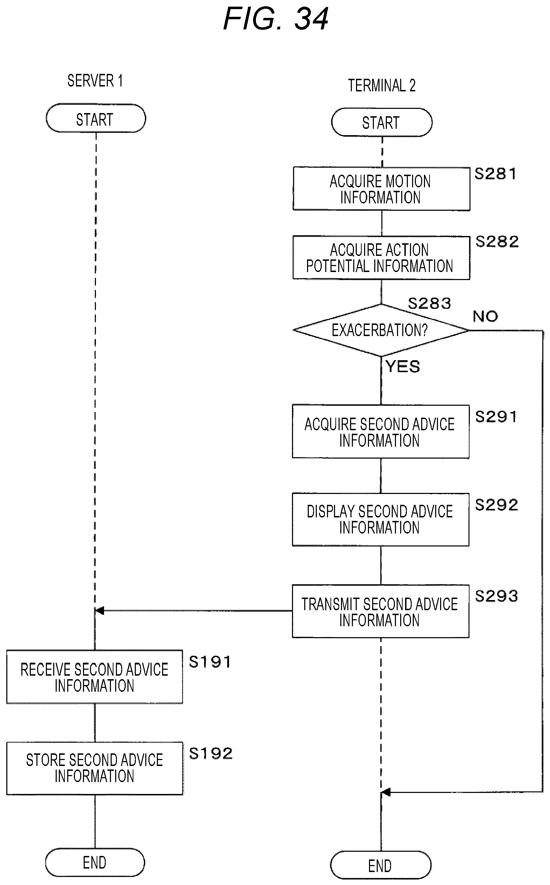

Fig. 34 is a flowchart illustrating a processing procedure of transmitting the second advice information according to the information relating to exacerbation. Meanwhile, content overlapping that of Fig. 33 is denoted by the same reference numerals and signs and thus description thereof will be omitted. In a case where it is determined that the information relating to exacerbation in COPD is detected in step S283, the control unit 21 of the terminal 2 acquires the second advice information according to the information relating to exacerbation (step S291). The second advice information is an opinion, a suggestion, or the like other than the medication instruction information described above, and may be useful information or the like for the treatment of COPD. The second advice information includes, for example, information relating to exercise therapy such as "Please walk" and "Please do basic training," or information relating to nutritional therapy such as "Be careful not to overeat," and "Please take care of your nutrition." Meanwhile, a process of acquiring the second advice information is the same as the process of acquiring the medication instruction information described above, and thus description thereof will not be given. The control unit 21 displays the acquired second advice information on the display unit 25 (step S292). The control unit 21 transmits the acquired second advice information to the server 1 through the communication unit 23 (step S293).

The control unit 11 of the server 1 receives the second advice information according to the information relating to exacerbation transmitted from the terminal 2 through the communication unit 13 (step S191), and stores the received second advice information in the medication instruction information DB 177 of the high-capacity storage unit 17 (step S192). Specifically, the control unit 11 allocates a medication instruction ID, and stores the patient ID, the information relating to exacerbation, and the second advice information as one record in the medication instruction information DB 177.

Meanwhile, the medication instruction information or the second advice information described above is transmitted to the terminal 2, but there is no limitation thereto. For example, the control unit 11 of the server 1 may transmit the medication instruction information together with the second advice information to the terminal 2 in accordance with the detected information relating to exacerbation.

In addition, after a patient has ingested a medication instruction medicine, information relating to medicine ingestion and measurement data after the medicine is ingested can be stored. Specifically, the control unit 11 acquires information relating to the ingestion of a medicine instructed to be taken by a patient from whom information relating to exacerbation has been detected through the communication unit 13 or the input unit 14. The control unit 11 acquires measurement data including motion information after medicine ingestion from the detection sensor 3, action potential information after medicine ingestion from the electromyogram sensor 4, and biological information after medicine ingestion from the second sensor 5 through the communication unit 13.

The control unit 11 stores the acquired information relating to medicine ingestion and measurement data after medicine ingestion in the treatment result DB 178 of the high-capacity storage unit 17. Specifically, the control unit 11 allocates a management ID, and stores the patient ID, the medication instruction ID, the medicine ingestion status, the motion information, the action potential information, and the biological information as one record in the treatment result DB 178.

Next, the process content of a process of re-learning the second exacerbation detection model 174 using the measurement data after medicine ingestion will be described. After a predetermined time has elapsed since a COPD patient ingested a medicine, the control unit 11 acquires second training data in which the measurement data including the motion information output from the detection sensor and the action potential information output from the electromyogram sensor and information indicating that exacerbation has not occurred (for example, level 1) are associated with each other. The control unit 11 re-learns the exacerbation detection model 173 (learned model) that outputs information relating to the presence or absence of exacerbation in a case where the motion information and the action potential information are input using the second training data. The control unit 11 stores the re-learned exacerbation detection model 173 in the high-capacity storage unit 17, and ends the series of processes.

In addition, it is possible to evaluate a patient after medicine ingestion and to take subsequent measures. For example, in a case where a patient who has been instructed to take a short-acting inhaled β2 stimulant with a bronchodilator effect has no improvement in the exacerbation condition such as dynamic hyperinflation even after he/she inhales the medicine, it is possible to take measures such as sending an instruction to visit a hospital to the patient.

According to the present embodiment, in a case where information relating to exacerbation in COPD is detected, it is possible to send the medication instruction information to a patient in accordance with the detected information relating to exacerbation.

According to the present embodiment, in a case where information relating to exacerbation in COPD is detected, it is possible to send the second advice information other than the medication instruction information to a patient in accordance with the detected information relating to exacerbation.

According to the present embodiment, it is possible to improve the accuracy of detection of information relating to exacerbation by re-learning the second exacerbation detection model using the measurement data after medicine ingestion.

### [Reference Signs List]

1 Information processing device (server)
11 Control unit
12 Storage unit
13 Communication unit
14 Input unit
15 Display unit
16 Reading unit
17 High-capacity storage unit
171 Patient DB
172 Master DB
173 Exacerbation detection model
174 Second exacerbation detection model
175 Advice DB
176 Walking model
177 Medication instruction information DB
178 Treatment result DB
1a Portable storage medium
1b Semiconductor memory
1P Control program
2 Information processing terminal (terminal)
21 Control unit
22 Storage unit
23 Communication unit
24 Input unit
25 Display unit
26 Bluetooth communication unit
2P Control program
3 Detection sensor
31 Control unit
32 Storage unit
33 Piezoelectric element
34 Bluetooth communication unit
3P Control program
4 Electromyogram sensor
41 Control unit
42 Storage unit
43 Myoelectric measurement unit
44 Bluetooth communication unit
4P Control program
5 Second sensor
6 Third sensor

## Claims

1. A computer program comprising instructions which, when the program is executed by a computer, cause the computer to execute the following processes:
• acquiring (S201) motion information as an electrical motion signal relating to motion of respiratory muscles or accessory respiratory muscles from a detection sensor (3) that detects the motion information;
• acquiring (S202) action potential information relating to the respiratory muscles or the accessory respiratory muscles from an electromyogram sensor (4) that acquires the action potential information;
• determining (S205) whether a first condition is satisfied on the basis of the acquired motion information;
• determining (S02, S04) whether the acquired action potential information corresponding to the expiratory muscles is equal to or higher than a predetermined threshold;
• outputting (S208) information relating to exacerbation in chronic obstructive pulmonary disease (COPD) in a case where the first condition is satisfied and the acquired action potential information corresponding to the expiratory muscles is equal to or higher than the predetermined threshold;
• determining that the first condition is satisfied in a case where a maximum value and/or a minimum value of the electrical motion signal which is output by the detection sensor continue/s to increase by a predetermined amount or more for a predetermined time;
• wherein the electrical motion signal is a voltage; and
• wherein said continuing increase is determined by:
∘ extracting the voltage maxima for the predetermined time in a time-series manner,
∘ calculating a slope of a first trend line formed by the extracted maxima, and
∘ if said slope exceeds a first predetermined slope threshold, determining that the first condition is satisfied, and/or
• wherein said continuing increase is determined by:
∘ extracting the voltage minima for the predetermined time in a time-series manner,
∘ calculating a slope of a second trend line formed by the extracted minima, and
∘ if said slope exceeds a second predetermined slope threshold, determining that the first condition is satisfied;
• wherein the first and second trend lines illustrate a temporal change in a voltage which is output from the detection sensor.

2. The program according to claim 1, wherein acquiring motion information relating to motion of respiratory muscles or accessory respiratory muscles from the detection sensor includes acquiring motion information relating to motion of respiratory muscles or accessory respiratory muscles from a piezoelectric element sensor, an extension sensor, an echo sensor, or a bioelectric potential sensor.

3. The program according to claim 1, wherein the predetermined threshold includes a first threshold and a second threshold larger than the first threshold, and
the program executes the following processes:
outputting (S03) information relating to exacerbation of a first level in a case where the acquired action potential information corresponding to the expiratory muscles is equal to or higher than the first threshold and less than the second threshold; and
outputting (S05) information relating to exacerbation of a second level in a case where the acquired action potential information corresponding to the expiratory muscles is equal to or higher than the second threshold.

4. The program according to any one of claims 1 to 3, executing the following processes:
acquiring (S241) biological information using a second sensor (5) that detects biological information of a patient; and
determining exacerbation in COPD from the acquired biological information, the motion information acquired by the detection sensor, and the action potential information acquired by the electromyogram sensor.

5. The program according to any one of claims 1 to 4, executing the following processes:
acquiring (S241) biological sound information including a respiratory sound, a cough sound, a sputum retention sound, or a pulmonary sound using a microphone; and
detecting exacerbation in COPD from the acquired biological sound information, the motion information, and the action potential information.

6. The program according to according to any one of claims 1 to 5, executing a process of acquiring (S264) activity information including an exercise amount, movement distance, activity amount, or posture of a patient using a third sensor (6) that detects the activity information.

7. The program according to claim 6, executing a process of, in a case where a predetermined activity is detected from the activity information acquired by the third sensor, changing the first condition for electrical motion signal in accordance with the detected activity, the predetermined threshold for the acquired action potential information corresponding to the expiratory muscles , or a predetermined threshold of biological information for determining biological information acquired by a second sensor (5).

8. The program according to claim 6 or 7, executing the following processes:
specifying (S265) activity content in accordance with the activity information;
acquiring (S162) advice information in accordance with the specified activity content, the motion information obtained from the detection sensor, the action potential information obtained from the electromyogram sensor, and the biological information obtained from the second sensor that detects biological information of a patient; and
transmitting (S163) the acquired advice information.

9. The program according to any one of claims 1 to 8, executing the following processes:
acquiring (S284) medication instruction information in accordance with the information relating to exacerbation; and
transmitting (S286) the acquired medication instruction information.

10. The program according to claim 1 or 2, executing the following processes:
acquiring (S201, S202) the motion information obtained from the detection sensor and the action potential information obtained from the electromyogram sensor;
inputting the acquired motion information and action potential information into a learning model (173) learned on the basis of a plurality of pieces of training data including the motion information, the action potential information, and the information relating to exacerbation in chronic obstructive pulmonary disease; and
outputting information relating to the presence or absence of exacerbation in chronic obstructive pulmonary disease.

11. The program according to claim 1 or 2, executing the following processes:
acquiring (S201, S202, S241) the motion information obtained from the detection sensor, the action potential information obtained from the electromyogram sensor, and biological information obtained from a second sensor (5) that detects biological information of a patient;
inputting the acquired motion information, action potential information, and biological information into a second learning model (174) learned on the basis of a plurality of pieces of training data including the motion information, the action potential information, the biological information, and information relating to exacerbation in chronic obstructive pulmonary disease; and
outputting information relating to the presence or absence of exacerbation in chronic obstructive pulmonary disease.

12. The program according to claim 10 or 11, executing the following processes:
specifying (S265) activity content in accordance with activity information including an exercise amount, movement distance, activity amount, or posture of a patient acquired by a third sensor (6) that detects the activity information;
selecting (S270) a learning model corresponding to activity information specified from a plurality of learning models prepared for each piece of activity content;
inputting the motion information and the action potential information into the selected learning model; and
outputting information relating to the presence or absence of exacerbation in chronic obstructive pulmonary disease.

13. The program according to any one of claims 10 to 12, executing the following processes:
acquiring motion information which is output from the detection sensor and action potential information which is output from the electromyogram sensor after elapse of a predetermined time from ingestion of medicine; and
re-learning a learned model using the acquired motion information and action potential information and information indicating that exacerbation in COPD does not occur.

14. An information processing device (1) comprising:
an acquisition unit configured to acquire motion information as an electrical motion signal relating to motion of respiratory muscles or accessory respiratory muscles from a detection sensor (3) that detects the motion information, and further configured to acquire action potential information relating to the respiratory muscles or the accessory respiratory muscles from an electromyogram sensor (4) that acquires the action potential information;
a control unit configured to determine whether a first condition is satisfied on the basis of the acquired motion information;
wherein the control unit is further configured to determine whether the acquired action potential information corresponding to the expiratory muscles is equal to or higher than a predetermined threshold; and
an output unit configured to output information relating to exacerbation in chronic obstructive pulmonary disease (COPD) in a case where the first condition is satisfied and the acquired action potential information corresponding to the expiratory muscles is equal to or higher than a predetermined threshold;
wherein the control unit is configured to determine that the first condition is satisfied in a case where a maximum value and/or a minimum value of the electrical motion signal which is output by the detection sensor continue/s to increase by a predetermined amount or more for a predetermined time; and
wherein the electrical motion signal is a voltage;
wherein the control unit is configured to determine said continuing increase by:
extracting the voltage maxima for the predetermined time in a time-series manner, calculating a slope of a first trend line formed by the extracted maxima, and if said slope exceeds a first predetermined slope threshold, determining that the first condition is satisfied, and/or
wherein the control unit is configured to determine said continuing increase by:
extracting the voltage minima for the predetermined time in a time-series manner, calculating a slope of a second trend line formed by the extracted minima, and if said slope exceeds a second predetermined slope threshold, determine that the first condition is satisfied;
wherein the first and second trend lines illustrate a temporal change in a voltage which is output from the detection sensor.

## Patentansprüche

1. Computerprogramm, umfassend Anweisungen, die, wenn das Programm von einem Computer ausgeführt wird, den Computer veranlassen, die folgenden Prozesse auszuführen:
• Erlangen (S201) von Bewegungsinformationen als ein elektrisches Bewegungssignal, das sich auf die Bewegung von der Atemmuskulatur oder der akzessorischen Atemmuskulatur bezieht, von einem Erfassungssensor (3), der die Bewegungsinformationen erfasst;
• Erlangen (S202) von Aktionspotentialinformationen, die sich auf die Atemmuskulatur oder die akzessorische Atemmuskulatur beziehen, von einem Elektromyogrammsensor (4), der die Aktionspotentialinformationen erfasst;
• Bestimmen (S205), ob eine erste Bedingung auf der Grundlage der erfassten Bewegungsinformationen erfüllt ist;
• Bestimmen (S02, S04), ob die erfassten Aktionspotentialinformationen, die der exspiratorischen Muskulatur entsprechen, gleich oder größer als ein vorbestimmter Schwellenwert sind;
• Ausgeben (S208) von Informationen, die sich auf eine Verschlimmerung der chronisch obstruktiven Lungenerkrankung (COPD) beziehen, in einem Fall, in dem die erste Bedingung erfüllt ist und die erfassten Aktionspotenzialinformationen, die der exspiratorischen Muskulatur entsprechen, gleich oder höher als der vorbestimmte Schwellenwert sind;
• Bestimmen, dass die erste Bedingung in einem Fall erfüllt ist, in dem ein Maximalwert und/oder ein Minimalwert des elektrischen Bewegungssignals, das von dem Erfassungssensor ausgegeben wird, fortfahren/fortfährt, für eine vorbestimmte Zeit um einen vorbestimmten Betrag oder mehr anzusteigen;
• wobei das elektrische Bewegungssignal eine Spannung ist; und
• wobei der anhaltende Anstieg bestimmt wird durch:
∘ Extrahieren der Spannungsmaxima für die vorbestimmte Zeit in Form einer Zeitserie,
∘ Berechnen einer Steigung einer ersten Trendlinie, die durch die extrahierten Maxima gebildet wird, und
∘ wenn die Steigung einen ersten vorbestimmten Steigungsschwellenwert überschreitet, Bestimmen, dass die erste Bedingung erfüllt ist, und/oder
• wobei der anhaltende Anstieg bestimmt wird durch:
o Extrahieren der Spannungsminima für die vorbestimmte Zeit in Form einer Zeitserie,
o Berechnen einer Steigung einer zweiten Trendlinie, die durch die extrahierten Minima gebildet wird, und
o wenn die Steigung einen zweiten vorbestimmten Steigungsschwellenwert überschreitet, Bestimmen; dass die erste Bedingung erfüllt ist;
• wobei die erste und die zweite Trendlinie eine zeitliche Änderung in einer Spannung veranschaulichen, die von dem Erfassungssensor ausgegeben wird.

2. Programm nach Anspruch 1, wobei das Erlangen von Bewegungsinformationen, die sich auf die Bewegung von der Atemmuskulatur oder der akzessorischen Atemmuskulatur beziehen, von dem Erfassungssensor das Erlangen von Bewegungsinformationen, die sich auf die Bewegung von der Atemmuskulatur oder der akzessorischen Atemmuskulatur von einem piezoelektrischen Sensorelement, einem Dehnungssensor, einem Echosensor oder einem bioelektrischen Potentialsensor beziehen, umfasst.

3. Programm nach Anspruch 1, wobei der vorbestimmte Schwellenwert einen ersten Schwellenwert und einen zweiten Schwellenwert, der größer als der erste Schwellenwert ist, umfasst, und das Programm die folgenden Prozesse ausführt:
Ausgeben (S03) von Informationen, die sich auf die Verschlimmerung einer ersten Stufe beziehen, in einem Fall, in dem die erfassten Aktionspotentialinformationen, die der exspiratorischen Muskulatur entsprechen, gleich oder höher als der erste Schwellenwert und kleiner als der zweite Schwellenwert sind; und
Ausgeben (S05) von Informationen, die sich auf die Verschlimmerung einer zweiten Stufe beziehen, in einem Fall, in dem die erste Bedingung erfüllt ist und die erfassten Aktionspotentialinformationen, die der exspiratorischen Muskulatur entsprechen, gleich oder höher als der vorbestimmte Schwellenwert sind.

4. Programm nach einem der Ansprüche 1 bis 3, das die folgenden Prozesse ausführt:
Erlangen (S241) biologischer Informationen unter Verwendung eines zweiten Sensors (5), der biologische Informationen eines Patienten erfasst; und
Bestimmen einer Verschlimmerung bei COPD aus den erfassten biologischen Informationen, den durch den Erfassungssensor erfassten Bewegungsinformationen und den durch den Elektromyogrammsensor erfassten Aktionspotentialinformationen.

5. Programm nach einem der Ansprüche 1 bis 4, das die folgenden Prozesse ausführt:
Erlangen (S241) von biologischen Geräuschinformationen, umfassend ein Atemgeräusch, ein Hustengeräusch, ein Sputumretentionsgeräusch oder ein Lungengeräusch unter Verwendung eines Mikrofons; und
Feststellen einer Verschlimmerung bei COPD aus den erfassten biologischen Geräuschinformationen, den Bewegungsinformationen und den Aktionspotentialinformationen.

6. Programm nach einem der Ansprüche 1 bis 5, für das Ausführen eines Prozesses zum Erfassen (S264) von Aktivitätsinformationen, umfassend einen Bewegungsbetrag, eine Bewegungsdistanz, einen Aktivitätsbetrag oder eine Körperhaltung eines Patienten, unter Verwendung eines dritten Sensors (6), der die Aktivitätsinformationen erfasst.

7. Programm nach Anspruch 6, für das Ausführen eines Prozesses, in einem Fall, bei dem eine vorbestimmte Aktivität aus den Aktivitätsinformationen, die von dem dritten Sensor erfasst, werden, festgestellt wird, zum Ändern von der ersten Bedingung für ein elektrisches Bewegungssignal in Übereinstimmung mit der erfassten Aktivität, dem vorbestimmten Schwellenwert für die erfassten Aktionspotentialinformationen, die der exspiratorischen Muskulatur entsprechen, oder einem vorbestimmten Schwellenwert für biologische Informationen zur Bestimmung biologischer Informationen, die von einem zweiten Sensor (5) erfasst werden.

8. Programm nach Anspruch 6 oder 7, das die folgenden Prozesse ausführt: Spezifizieren (S265) des Aktivitätsinhalts in Übereinstimmung mit den Aktivitätsinformationen;
Erlangen (S162) von Empfehlungsinformationen in Übereinstimmung mit dem spezifizierten Aktivitätsinhalt, der vom Erfassungssensor erhaltenen Bewegungsinformationen, der vom Elektromyogrammsensor erhaltenen Aktionspotentialinformationen und der vom zweiten Sensor, der die biologischen Informationen eines Patienten erfasst, erhaltenen biologischen Informationen; und
Übertragen (S163) der erlangten Empfehlungsinformationen.

9. Programm nach einem der Ansprüche 1 bis 8, das die folgenden Prozesse ausführt:
Erlangen (S284) von Medikationsanweisungsinformationen in Übereinstimmung mit den Informationen, die sich auf die Verschlimmerung beziehen; und
Übertragen (S286) der erlangten Medikationsanweisungsinformationen.

10. Programm nach Anspruch 1 oder 2, das die folgenden Prozesse ausführt:
Erlangen (S201, S202) der Bewegungsinformationen, die von dem Erfassungssensor erhalten wurden, und der Aktionspotentialinformationen,
die von dem Elektromyogrammsensor erhalten wurden;
Eingeben der erfassten Bewegungsinformationen und Aktionspotentialinformationen in ein Lernmodell (173), das auf der Grundlage einer Vielzahl von Teilen der Trainingsdaten erlernt wurde, welche die Bewegungsinformationen, die Aktionspotentialinformationen und die Informationen in Bezug auf eine Verschlimmerung der chronisch obstruktiven Lungenerkrankung umfassen; und
Ausgeben von Informationen in Bezug auf das Vorhandensein oder Nichtvorhandensein einer Verschlimmerung der chronisch obstruktiven Lungenerkrankung.

11. Programm nach Anspruch 1 oder 2, das die folgenden Prozesse ausführt:
Erlangen (S201, S202, S241) von Bewegungsinformationen, die von dem Erfassungssensor erhalten werden, von Aktionspotentialinformationen, die von dem Elektromyogrammsensor erhalten werden, und von biologischen Informationen, die von einem zweiten Sensor (5) erhalten werden, der die biologischen Informationen eines Patienten erfasst;
Eingeben der erfassten Bewegungsinformationen, der Aktionspotentialinformationen und der biologischen Informationen in ein zweites Lernmodell (174), das auf der Grundlage einer Vielzahl von Teilen der Trainingsdaten erlernt wurde, welche die Bewegungsinformationen, die Aktionspotentialinformationen, die biologischen Informationen und die Informationen in Bezug auf eine Verschlimmerung der chronisch obstruktiven Lungenerkrankung umfassen; und
Ausgeben von Informationen in Bezug auf das Vorhandensein oder Nichtvorhandensein einer Verschlimmerung der chronisch obstruktiven Lungenerkrankung.

12. Programm nach Anspruch 10 oder 11, das die folgenden Prozesse ausführt:
Spezifizieren (S265) eines Aktivitätsinhalts in Übereinstimmung mit Aktivitätsinformationen, die einen Bewegungsbetrag, eine Bewegungsdistanz, einen Aktivitätsbetrag oder eine Körperhaltung eines Patienten umfassen, die von einem dritten Sensor (6) erfasst werden, der die Aktivitätsinformationen detektiert;
Auswählen (S270) eines Lernmodells, das den spezifizierten Aktivitätsinformationen entspricht, aus einer Vielzahl von Lernmodellen, die für jedes Teil des Aktivitätsinhalts aufgestellt wurden;
Eingeben der Bewegungsinformationen und der Aktionspotentialinformationen in das ausgewählte Lernmodell; und
Ausgeben von Informationen in Bezug auf das Vorhandensein oder Nichtvorhandensein einer Verschlimmerung der chronisch obstruktiven Lungenerkrankung.

13. Programm nach einem der Ansprüche 10 bis 12, das die folgenden Prozesse ausführt:
Erlangen von Bewegungsinformationen, die von dem Erfassungssensor ausgegeben werden, und von Aktionspotentialinformationen, die von dem Elektromyogrammsensor ausgegeben werden, nach Ablauf einer vorbestimmten Zeit nach der Einnahme eines Medikaments; und
erneutes Lernen eines erlernten Modells unter Verwendung der erfassten Bewegungsinformationen und Aktionspotentialinformationen und von Informationen, die anzeigen, dass eine Verschlimmerung bei COPD nicht auftritt.

14. Informationsverarbeitungsvorrichtung (1) umfassend:
eine Erfassungseinheit, die so konfiguriert ist, dass sie
Bewegungsinformationen als ein elektrisches Bewegungssignal, das sich auf die Bewegung von der Atemmuskulatur oder der akzessorischen Atemmuskulatur bezieht, von einem Erfassungssensor (3) erfasst, welcher die Bewegungsinformationen erfasst, und die ferner so konfiguriert ist, dass sie Aktionspotentialinformationen, die sich auf die Atemmuskulatur oder die akzessorische Atemmuskulatur beziehen, von einem Elektromyogrammsensor (4) erfasst, der die Aktionspotentialinformationen erfasst;
eine Steuerungseinheit, die so konfiguriert ist, dass sie auf der Grundlage der erfassten Bewegungsinformationen feststellt, ob eine erste Bedingung erfüllt ist;
wobei die Steuerungseinheit ferner so konfiguriert ist, dass sie feststellt, ob die erfassten Aktionspotentialinformationen, die der exspiratorischen Muskulatur entsprechen, gleich oder höher als ein vorgegebener Schwellenwert sind; und
eine Ausgabeeinheit, die so konfiguriert ist, dass sie in einem Fall, in dem die erste Bedingung erfüllt ist und die erfasste Aktionspotentialinformation, die den Ausatmungsmuskulatur entspricht, gleich oder höher als ein vorbestimmter Schwellenwert ist, Informationen ausgibt, die sich auf eine Verschlimmerung der chronisch obstruktiven Lungenerkrankung (COPD) beziehen;
wobei die Steuerungseinheit so konfiguriert ist, dass sie bestimmt, dass die erste Bedingung in einem Fall erfüllt ist, in dem ein Maximalwert und/oder ein Minimalwert des elektrischen Bewegungssignals, das von dem Erfassungssensor ausgegeben wird, fortfahren/fortfährt, für eine vorbestimmte Zeit um einen vorbestimmten Betrag oder mehr anzusteigen; und
wobei das elektrische Bewegungssignal eine Spannung ist;
wobei die Steuerungseinheit so konfiguriert ist, dass sie den anhaltenden Anstieg bestimmt durch:
Extrahieren der Spannungsmaxima für die vorbestimmte Zeit in Form einer Zeitserie, Berechnen einer Steigung einer ersten Trendlinie, die durch die extrahierten Maxima gebildet wird, und wenn die Steigung einen ersten vorbestimmten Steigungsschwellenwert überschreitet, Bestimmen, dass die erste Bedingung erfüllt ist, und/oder
wobei die Steuerungseinheit so konfiguriert ist, dass sie den anhaltenden Anstieg bestimmt durch:
Extrahieren der Spannungsminima für die vorbestimmte Zeit in Form einer Zeitserie, Berechnen einer Steigung einer zweiten Trendlinie, die durch die extrahierten Minima gebildet wird, und wenn die Steigung einen zweiten vorbestimmten Steigungsschwellenwert überschreitet, Bestimmen, dass die erste Bedingung erfüllt ist;
wobei die erste und die zweite Trendlinie eine zeitliche Änderung in einer Spannung veranschaulichen, die von dem Erfassungssensor ausgegeben wird.

## Revendications

1. Programme informatique comprenait des instructions qui, lorsque le programme est exécuté par un ordinateur, font que l'ordinateur exécute les processus suivants :
- acquérir (S201) des informations de mouvement en tant qu'un signal de mouvement électrique lié au mouvement de muscles respiratoires ou muscles respiratoires accessoires venant d'un capteur de détection (3) qui détecte les informations de mouvement ;
- acquérir (S202) des informations de potentiel d'action liées aux muscles respiratoires ou aux muscles respiratoires accessoires venant d'un capteur d'électromyogramme (4) qui acquiert les informations de potentiel d'action ;
- déterminer (S205) si une première condition est satisfaite en se basant sur les informations de mouvement acquises ;
- déterminer (S02, S04) si les informations de potentiel d'action acquises correspondant aux muscles expiratoires sont supérieures ou égales à un seuil prédéterminé ;
- émettre (S208) des informations liées à l'exacerbation dans la maladie pulmonaire obstructive chronique (COPD) dans un cas où la première condition est satisfaite et les informations de potentiel d'action acquises correspondant aux muscles expiratoires sont supérieures ou égales au seuil prédéterminé ;
- déterminer que la première condition est satisfaite dans un cas où une valeur maximale et/ou une valeur minimale du signal de mouvement électrique qui est émis par le capteur de détection continue(nt) d'augmenter d'une quantité prédéterminée ou plus pendant un temps prédéterminé ;
- dans lequel le signal de mouvement électrique est une tension ; et
- dans lequel ladite augmentation continue est déterminée par :
- l'extraction des maximums de tension pendant la durée prédéterminée d'une manière temps-série,
- le calcul d'une pente d'une première ligne de tendance formée par les maximums extraits, et
- si ladite pente dépasse un premier seuil de pente prédéterminé, déterminer que la première condition est satisfaite, et/ou
- dans lequel ladite augmentation continue est déterminée par :
- l'extraction des minimums de tension pendant la durée prédéterminée d'une manière temps-série,
- le calcul d'une pente d'une seconde ligne de tendance formée par les minimums extraits, et
- si ladite pente dépasse un second seuil de pente prédéterminé, déterminer que la première condition est satisfaite ;
- dans lequel les première et seconde lignes de tendance illustrent un changement temporel dans une tension qui est émise par le capteur de détection.

2. Programme selon la revendication 1, dans lequel acquérir des informations de mouvement liées au mouvement de muscles respiratoires ou de muscles respiratoires accessoires venant du capteur de détection inclut d'acquérir des informations de mouvement liées au mouvement de muscles respiratoires ou de muscles respiratoires accessoires d'un capteur d'élément piézoélectrique, d'un capteur d'extension, d'un capteur d'écho ou d'un capteur de potentiel bioélectrique.

3. Programme selon la revendication 1, dans lequel le seuil prédéterminé inclut un premier seuil et un second seuil plus grand que le premier seuil, et le programme exécute les processus suivants :
émettre (S03) des informations liées à l'exacerbation d'un premier niveau dans un cas où les informations de potentiel d'action acquises correspondant aux muscles expiratoires sont supérieures ou égales au premier seuil et inférieures ou égales au second seuil ; et
émettre (S05) des informations liées à l'exacerbation d'un second niveau dans un cas où les informations de potentiel d'action acquises correspondant aux muscles expiratoires sont supérieures ou égales au second seuil.

4. Programme selon l'une quelconque des revendications 1 à 3, exécutant les processus suivants :
acquérir (S241) des informations biologiques en utilisant un deuxième capteur (5) qui détecte les informations biologiques d'un patient ; et
déterminer l'exacerbation dans la COPD à partir des informations biologiques acquises, des informations de mouvement acquises par le capteur de détection et des informations de potentiel d'action acquises par le capteur d'électromyogramme.

5. Programme selon l'une quelconque des revendications 1 à 4, exécutant les processus suivants :
acquérir (S241) des informations de son biologique incluant un son respiratoire, un son de toux, un son de rétention d'expectoration ou un son pulmonaire en utilisant un microphone ; et
détecter l'exacerbation dans la COPD à partir des informations biologiques acquises, des informations de mouvement et des informations de potentiel d'action.

6. Programme selon l'une quelconque des revendications 1 à 5, exécutant un processus d'acquisition (S264) des informations d'activité incluant une quantité d'exercices, une distance de mouvement, une quantité d'activité ou une posture d'un patient en utilisant un troisième capteur (6) qui détecte les informations d'activité.

7. Programme selon la revendication 6, exécutant un processus, dans un cas où une activité prédéterminée est détectée à partir des informations d'activité acquises par le troisième capteur, de changement de la première condition pour le signal de mouvement électrique selon l'activité détectée, le seuil prédéterminé pour les informations de potentiel d'action acquises correspondant aux muscles expiratoires ou un seuil prédéterminé d'informations biologiques pour déterminer des informations biologiques acquises par un deuxième capteur (5).

8. Programme selon la revendication 6 ou 7, exécutant les processus suivants :
spécifier (S265) un contenu d'activités selon les informations d'activité ;
acquérir (S162) des informations de conseil selon le contenu d'activité spécifié, les informations de mouvement obtenues du capteur de détection, les informations de potentiel d'action obtenues du capteur d'électromyogramme et les informations biologiques obtenues du deuxième capteur qui détecte les informations biologiques d'un patient ; et
transmettre (S163) les informations de conseil acquises.

9. Programme selon l'une quelconque des revendications 1 à 8, exécutant les processus suivants :
acquérir (S284) des informations d'instruction de médicaments selon les informations relatives à l'exacerbation ; et
transmettre (S286) les informations d'instruction de médicament acquises.

10. Programme selon la revendication 1 ou 2, exécutant les processus suivants :
acquérir (S201, S202) les informations de mouvement obtenues du capteur de détection et les informations de potentiel d'action obtenues du capteur d'électromyogramme ;
saisir les informations de mouvement acquises et les informations de potentiel d'action dans un modèle d'apprentissage (173) appris en se basant sur une pluralité de données de formation incluant les informations de mouvement, les informations de potentiel d'action et les informations liées à l'exacerbation dans la maladie pulmonaire obstructive chronique ; et
émettre des informations relatives à la présence ou à l'absence d'exacerbation dans la maladie pulmonaire obstructive chronique.

11. Programme selon la revendication 1 ou 2, exécutant les processus suivants :
acquérir (S201, S202, S241) les informations de mouvement obtenues du capteur de détection, les informations de potentiel d'action obtenues du capteur d'électromyogramme et les informations biologiques obtenues d'un second capteur (5) qui détecte des informations biologiques d'un patient ;
saisir les informations de mouvement acquises, les informations de potentiel d'action et les informations biologiques dans un second modèle d'apprentissage (174) appris en se basant sur une pluralité de données de formation incluant les informations de mouvement, les informations de potentiel d'action, les informations biologiques et les informations liées à l'exacerbation dans la maladie pulmonaire obstructive chronique ; et
émettre des informations relatives à la présence ou à l'absence d'exacerbation dans la maladie pulmonaire obstructive chronique.

12. Programme selon la revendication 10 ou 11, exécutant les processus suivants :
spécifier (S265) un contenu d'activité selon les informations d'activité incluant une quantité d'exercices, une distance de mouvement, une quantité d'activité ou une posture d'un patient acquise par un troisième capteur (6) qui détecte les informations d'activité ;
sélectionner (S270) un modèle d'apprentissage correspondant à des informations d'activité spécifiées à partir d'une pluralité de modèles d'apprentissage préparés pour chaque contenu d'activité ;
saisir les informations de mouvement et les informations de potentiel d'action dans le modèle d'apprentissage sélectionné ; et
émettre des informations relatives à la présence ou à l'absence d'exacerbation dans la maladie pulmonaire obstructive chronique.

13. Programme selon l'une quelconque des revendications 10 à 12, exécutant les processus suivants :
acquérir des informations de mouvement qui sont émises par le capteur de détection et des informations de potentiel d'action qui sont émises par le capteur d'électromyogramme après écoulement d'un temps prédéterminé d'ingestion d'un médicament ; et
ré-apprentissage d'un modèle appris en utilisant les informations de mouvement et les informations de potentiel d'action acquises et les informations indiquant que l'exacerbation dans la COPD ne se produit pas.

14. _ Dispositif de traitement d'informations (1) comprenant :
une unité d'acquisition configurée pour acquérir des informations de mouvement en tant qu'un signal de mouvement électrique lié au mouvement de muscles respiratoires ou muscles respiratoires accessoires venant d'un capteur de détection (3) qui détecte les informations de mouvement, et configuré en outre pour acquérir des informations de potentiel d'action liées aux muscles respiratoires ou aux muscles respiratoires accessoires venant d'un capteur d'électromyogramme (4) qui acquiert les informations de potentiel d'action ;
une unité de commande configurée pour déterminer si une première condition est satisfaite en se basant sur les informations de mouvement acquises ;
dans lequel l'unité de commande est en outre configurée pour déterminer si les informations de potentiel d'action acquises correspondant aux muscles expiratoires sont supérieures ou égales à un seuil prédéterminé ; et
une unité d'émission configurée pour émettre des informations liées à l'exacerbation dans la maladie pulmonaire obstructive chronique (COPD) dans un cas où la première condition est satisfaite et les informations de potentiel d'action acquises correspondant aux muscles expiratoires sont supérieures ou égales à un seuil prédéterminé ;
dans lequel l'unité de commande est configurée pour déterminer que la première condition est satisfaite dans un cas où une valeur maximale et/ou une valeur minimale du signal de mouvement électrique qui est émis par le capteur de détection continue(nt) d'augmenter d'une quantité prédéterminée ou plus pendant un temps prédéterminé ;
dans lequel le signal de mouvement électrique est une tension ;
dans lequel l'unité de commande est configurée pour déterminer ladite augmentation continue par :
l'extraction des maximums de tension pendant la durée prédéterminée d'une manière temps-série, le calcul d'une pente d'une première ligne de tendance formée par les maximums extraits, et si ladite pente dépasse un premier seuil de pente prédéterminé, de déterminer que la première condition est satisfaite, et/ou
dans lequel l'unité de commande est configurée pour déterminer ladite augmentation continue par :
l'extraction des minimums de tension pendant la durée prédéterminée d'une manière temps-série, le calcul d'une pente d'une seconde ligne de tendance formée par les minimums extraits, et si ladite pente dépasse un second seuil de pente prédéterminé, déterminer que la première condition est satisfaite ;
dans lequel les première et seconde lignes de tendance illustrent un changement temporel dans une tension qui est émise par le capteur de détection.
